# EUROPEAN PATENT SPECIFICATION

(11) **EP 4 010 045 B1**
(45) Date of publication and mention of the grant of the patent: **20.03.2024**
(21) Application number: 20754489.1
(22) Date of filing: 23.07.2020
(51) Int. Cl.: A61M 60/135, A61M 60/268, A61M 60/295, A61M 60/427, A61M 60/497, A61M 60/508, A61M 60/835, A61M 60/841, A61M 60/857, A61M 60/859, A61M 60/861, A61M 60/869, A61M 60/882, A61M 60/896

(54) **FLUID VESSEL PUMP**
FLUIDBEHÄLTERPUMPE
POMPE À RÉSERVOIR DE FLUIDE

(30) Priority: 05.08.2019 US 201962882754 P
(43) Date of publication of application: 15.06.2022
(73) Proprietor: Edwards Lifesciences Corporation, Irvine, CA 92614-5688 (US)
(72) Inventor: MANASH, Boaz, 3052602 Givat Ada (IL); KVELER, Marat, 30889 Caesarea (IL)
(74) Representative: Eisenführ Speiser
(86) International application number: PCT/US2020/043214
(87) International publication number: WO 2021/025870

(56) References cited:
- WO-A1-2019/071148
- US-A1- 2002 103 413
- US-A1- 2016 015 877
- US-B2- 9 125 567

## Description

### CROSS-REFERENCE TO RELATED APPLICATIONS

This application claims priority to U.S. Provisional Application No. 62/882,754, filed August 5, 2019 and entitled "Fluid Vessel Pump".

### BACKGROUND

### Field

The present disclosure relates to the field of medical devices and procedures.

### Description of the Related Art

Congestive heart failure (CHF) is a disorder in which the heart fails to pump blood adequately to other organs in the body. CHF is a progressive condition that may cause the heart muscle to weaken or stiffen over time leading to a reduction in cardiac output and may exacerbate symptoms of heart failure. In some CHF cases, systemic fluid congestion, which can lead to CHF, results from right-sided heart failure. For example, the right-side of the heart may be unable to efficiently return blood back to the heart, such as through the vena cava. This may lead to shortness of breath, exercise intolerance, fatigue, elevated venous pressure, edema, hospitalization, or death.

Relevant prior art is for instance disclosed in documents US9125567 B2, US2002/103413 A1, US2016/015877 A1 and WO2019/071148 A1.

### SUMMARY

An inter-vessel fluid pump according to the present invention comprises the technical features as defined in independent claim 1. A vessel pump according to the present invention comprises the technical features as defined in independent claim 10.

Described herein are one or more methods (not claimed) and/or devices to facilitate an increase in fluid flow through use of one or more expandable chamber structures that are implantable in certain vessels and/or chambers of the human anatomy.

In some implementations, the present disclosure relates to an inter-vessel fluid pump comprising a first expandable chamber structure configured to be implanted within a first fluid vessel; and a second expandable chamber structure configured to be implanted within a second fluid vessel that is adjacent to the first fluid vessel. The second expandable chamber structure extends longitudinally and is in fluid communication with the first expandable chamber structure. In some implementations, compression of the first expandable chamber structure causes expansion of the second expandable chamber structure.

In some embodiments, the inter-vessel fluid pump further comprises a first conduit structure having an hour-glass-shaped profile and a lumen that extends longitudinally and a second conduit structure extending longitudinally and including one or more holes that extend radially. The second conduit structure is configured to house at least a portion of the first conduit structure and to align longitudinally with the first conduit structure. The second expandable chamber structure is configured to be disposed between the first conduit structure and the second conduit structure.

In some embodiments, the inter-vessel fluid pump further comprises a valve structure configured to align longitudinally with the first conduit structure and configured to be disposed between the second expandable chamber structure and the second conduit structure. The valve structure is configured to be displaced radially to obstruct the one or more holes of the second conduit structure when the second expandable chamber structure is expanded. In some embodiments, one or more of the second expandable chamber structure and the valve structure has a tapered cylindrical shape. In some embodiments, the first conduit structure includes one or more one-way valves disposed in at least one end of the first conduit structure. In some embodiments, in a deployed state: the first conduit structure and the second conduit structure are disposed to create a suction cavity between the first conduit structure and the second conduit structure, and the second expandable chamber structure is configured to expand radially to push a fluid in the suction cavity through the one or more one-way valves.

In some embodiments, one or more of the first expandable chamber structure and the second expandable chamber structure includes a wire-frame structure. In some embodiments, the inter-vessel fluid pump further comprises a conduit structure to connect the first expandable chamber structure to the second expandable chamber structure with the fluid.

In some implementations, the present disclosure relates to a method (not claimed) of increasing diastolic pressure comprises deploying a first expandable chamber structure in an aorta of a patient, and deploying a second expandable chamber structure in an inferior vena cava of the patient. The second expandable chamber structure is in fluid communication with the first expandable chamber structure via a conduit structure that extends through a wall in the aorta and a wall in the inferior vena cava.

In some embodiments, deploying the second expandable chamber structure includes deploying the second expandable chamber structure in the inferior vena cava to axially overlap at least a portion of an input of a renal vein into the inferior vena cava. In some embodiments, the method further comprises pumping, using the first expandable chamber structure and the second expandable chamber structure, blood from the renal vein into the inferior vena cava. In some embodiments, the pumping includes drawing blood from the renal vein into a suction cavity associated with the second expandable chamber structure during diastole and pumping the blood from the suction cavity during systole. In some embodiments, drawing the blood into the suction cavity includes expanding the first expandable chamber structure and collapsing the second expandable chamber structure, and pumping the blood from the suction cavity includes collapsing the first expandable chamber structure and expanding the second expandable chamber structure.

In some embodiments, the method further comprises advancing a guidewire through the wall of the inferior vena cava and the wall of the aorta at a location that is within a distance to a renal vein in the inferior vena cava. Deploying the first expandable chamber structure in the aorta includes advancing, using the guidewire, a delivery catheter through the inferior vena cava into the aorta.

In some implementations, the present disclosure relates to a vessel pump comprising a first expandable chamber structure configured to be implanted within a first fluid vessel, a second expandable chamber structure that is configured to be in fluid communication with the first expandable chamber structure, and an anchor structure configured to be implanted within a second fluid vessel and to house at least a portion of the second expandable chamber structure. In a deployed state, the second expandable chamber structure is configured to expand to fill at least a portion of a suction cavity within the anchor structure.

In some embodiments, the first fluid vessel comprises an abdominal aorta and the second fluid vessel comprises an inferior vena cava. In some embodiments, a cross-section of a first end of the anchor structure has a first diameter and a cross-section of a central portion of the anchor structure has a second diameter that is smaller than the first diameter. In some embodiments, one or more of the first expandable chamber structure and the second expandable chamber structure comprise a compliant balloon.

In some embodiments, the anchor structure includes one or more one-way valves configured to permit flow of fluid from the suction cavity into the second fluid vessel when deployed. In some embodiments, the vessel pump further comprises a valve structure configured to be disposed within with the anchor structure and to be displaced radially to obstruct one or more holes in the anchor structure when the second expandable chamber structure is expanded.

For purposes of summarizing the disclosure, certain aspects, advantages and novel features have been described. It is to be understood that not necessarily all such advantages may be achieved in accordance with any particular embodiment. Thus, the disclosed embodiments may be carried out in a manner that achieves or optimizes one advantage or group of advantages as taught herein without necessarily achieving other advantages as may be taught or suggested herein.

### BRIEF DESCRIPTION OF THE DRAWINGS

Various embodiments are depicted in the accompanying drawings for illustrative purposes and should in no way be interpreted as limiting the scope of the disclosure. In addition, various features of different disclosed embodiments can be combined to form additional embodiments, which are part of this disclosure. Throughout the drawings, reference numbers may be reused to indicate correspondence between reference elements.
Figure 1 illustrates example human anatomy having various features relevant to certain aspects of the present disclosure, wherein an example fluid vessel pump is implanted therein.
Figure 2 illustrates an example device that includes a first expandable chamber structure and an anchor structure to pump fluid in accordance with one or more embodiments.
Figure 3 illustrates a cross-sectional view of an aorta and an inferior vena cava with an example device implanted therein to pump blood in accordance with one or more embodiments.
Figure 4-1 illustrates an example upper portion of a conduit structure in accordance with one or more embodiments.
Figure 4-2 illustrates an example lower portion of a conduit structure in accordance with one or more embodiments.
Figure 4-3 illustrates an example conduit structure that is configured to associate with another conduit structure in accordance with one or more embodiments.
Figure 4-4 illustrates an example expandable chamber structure that is configured to be implemented with one or more conduit structures in accordance with one or more embodiments.

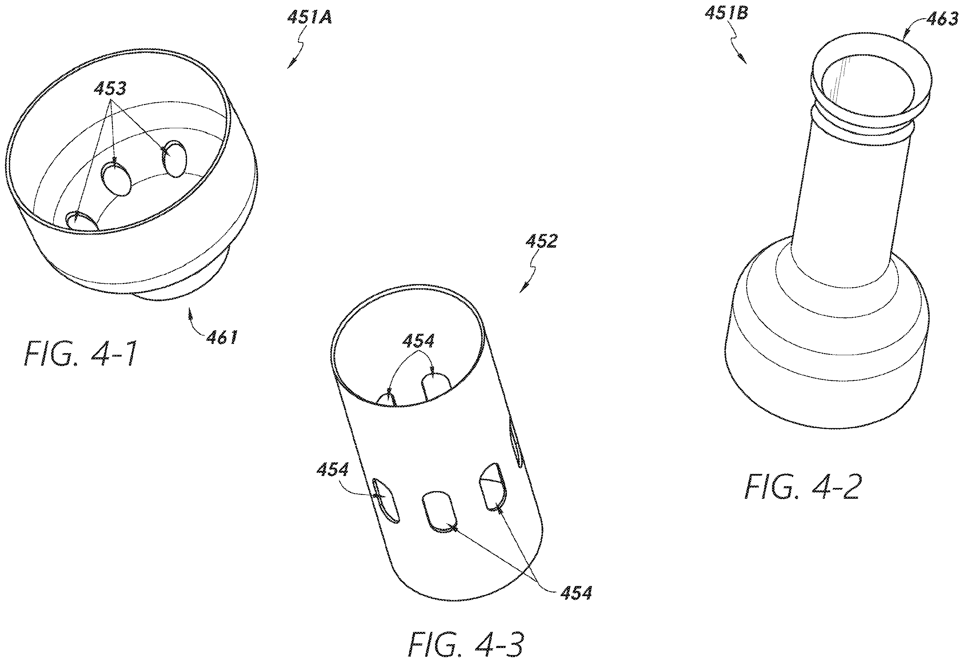

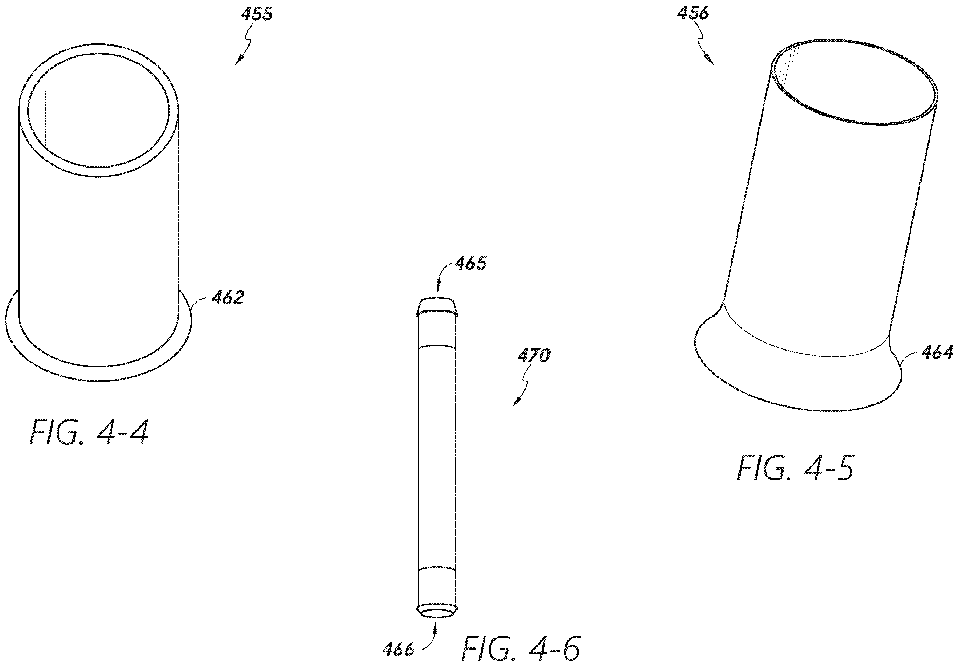

Figure 4-5 illustrates an example valve structure is configured to be implemented with one or more conduit structures and an expandable chamber structure in accordance with one or more embodiments.
Figure 4-6 illustrates an example expandable chamber structure that is configured to be connected to another expandable chamber structure in accordance with one or more embodiments.

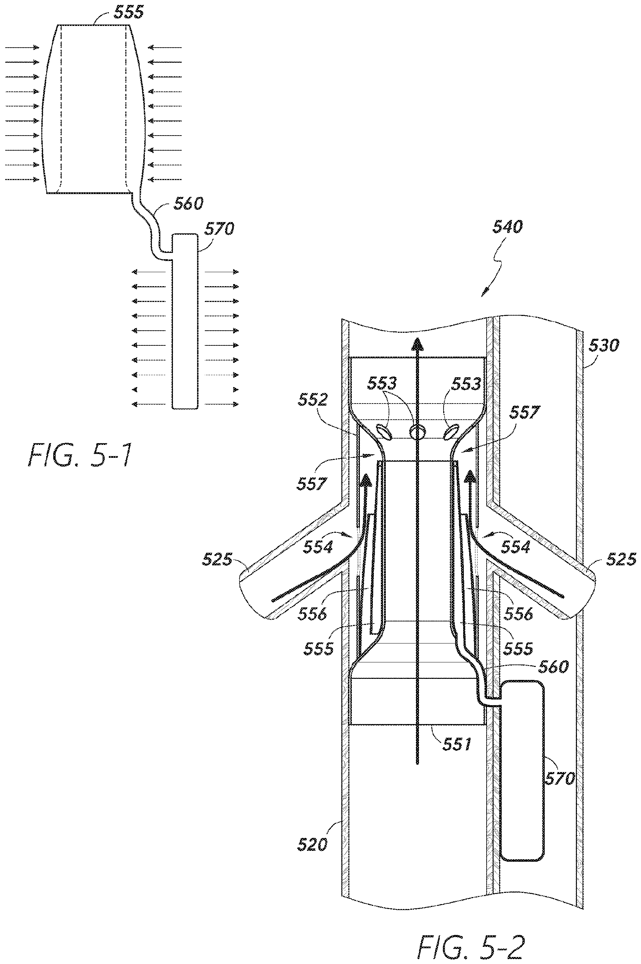

Figure 5-1 illustrates an example device that pumps blood during a diastole phase of a cardiac cycle in accordance with one or more embodiments.
Figure 5-2 illustrates an example device within a human anatomy during a diastole phase in accordance with one or more embodiments.

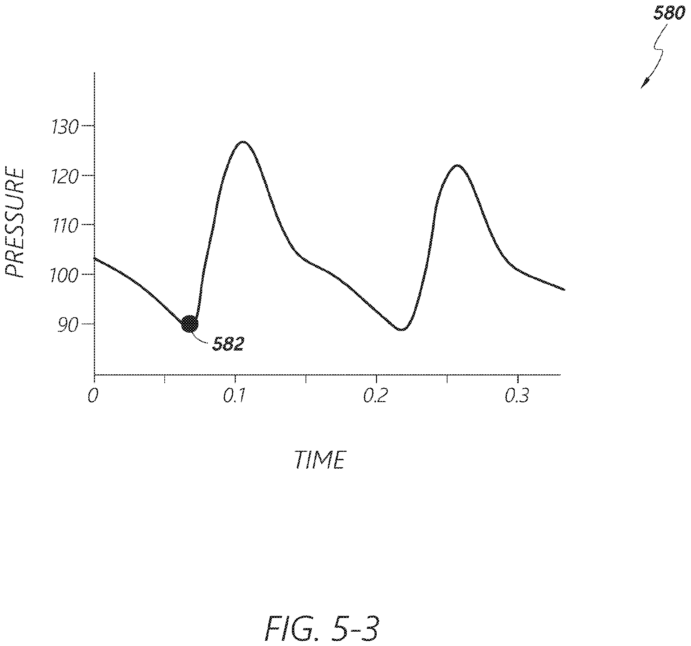

Figure 5-3 illustrates a graph of aortic pressure and a point when aortic pressure is at minimum in accordance with one or more embodiments.

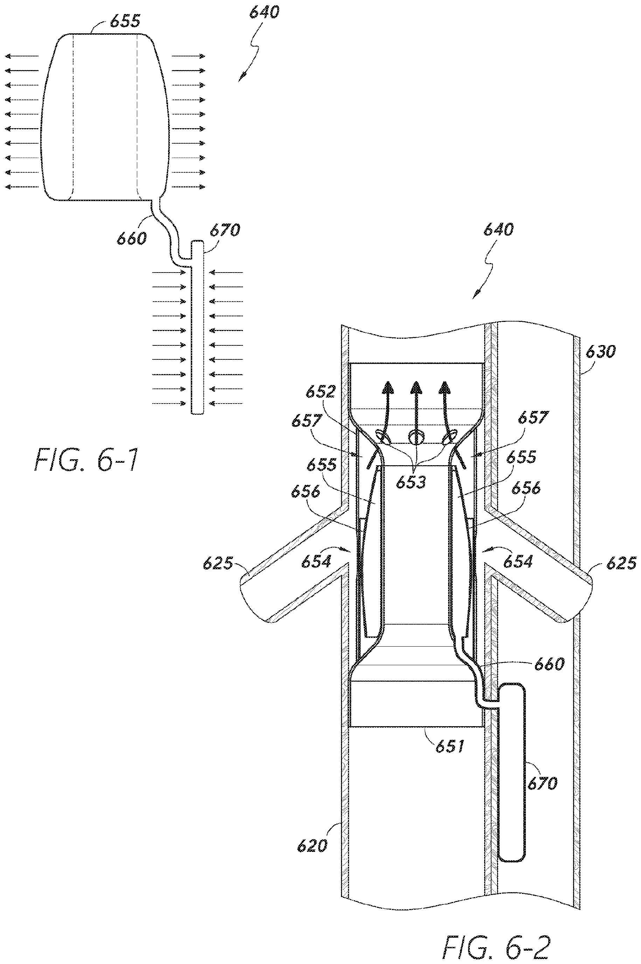

Figure 6-1 illustrates an example device that pumps blood during a systole phase of a cardiac cycle in accordance with one or more embodiments.
Figure 6-2 illustrates an example device within a human anatomy during a systole phase in accordance with one or more embodiments.

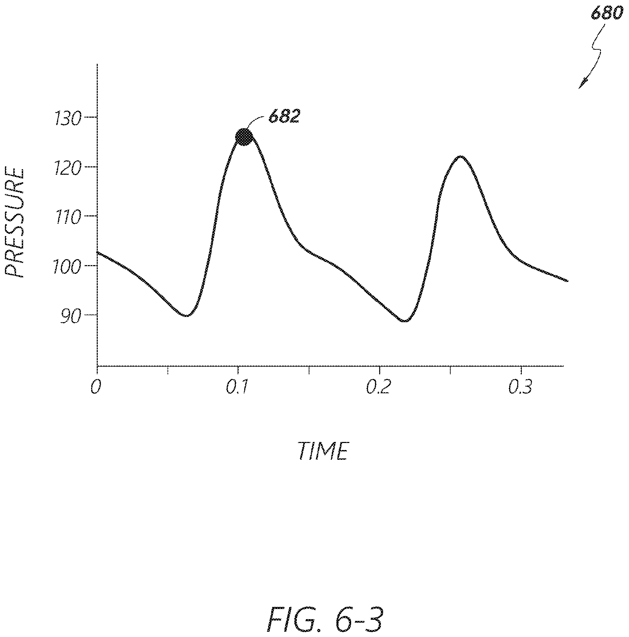

Figure 6-3 illustrates a graph of aortic pressure and a point when aortic pressure is at maximum in accordance with one or more embodiments.

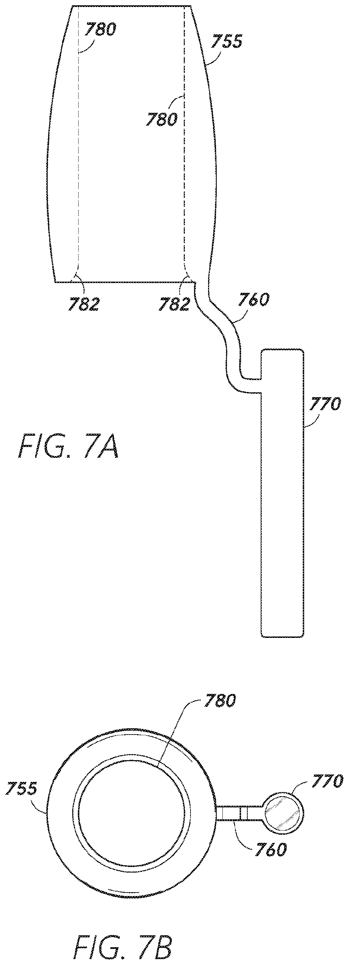

Figure 7A illustrates a side view of an expandable chamber structure connected to another expandable chamber structure via a conduit structure in accordance with one or more embodiments.
Figure 7B illustrates a top view of the expandable chamber structures of Figure 7A.

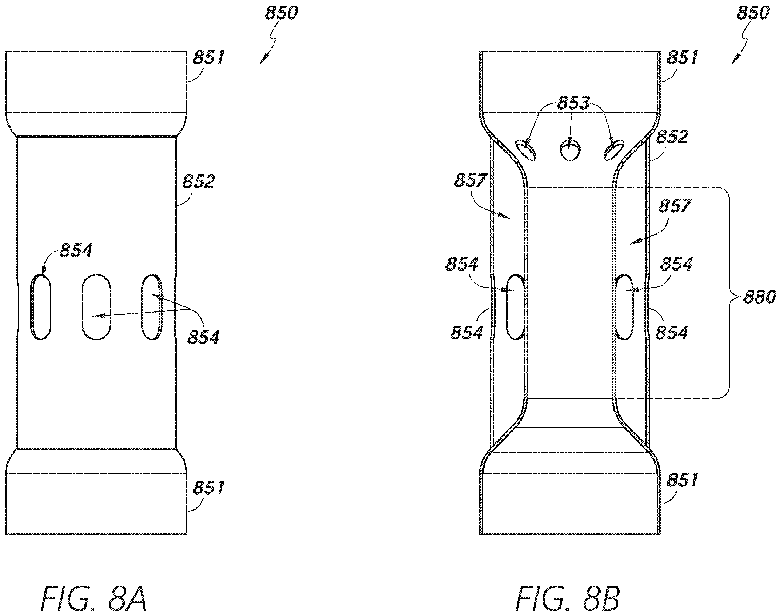

Figure 8A illustrates a side view of an example anchor structure that includes conduit structures in an attached configuration and that can be implemented in one or more embodiments.
Figure 8B illustrates a cross-sectional view of the anchor structure of Figure 8A in accordance with one or more embodiments.

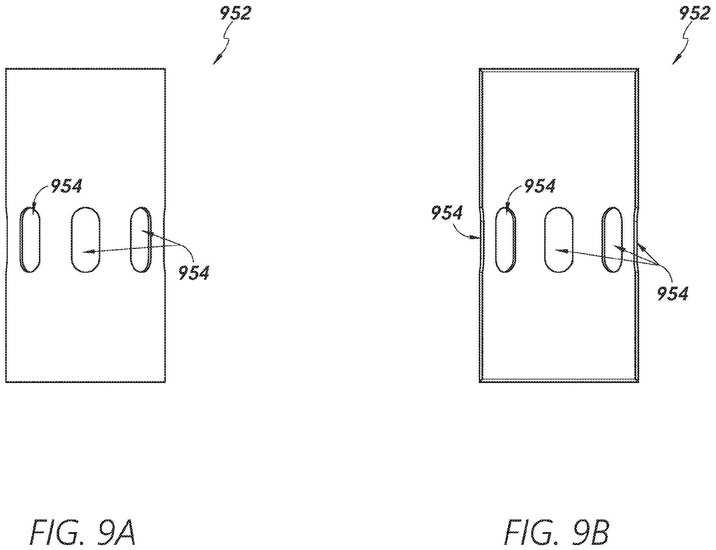

Figure 9A illustrates a side view of an example conduit structure that has a substantially cylindrical form and that can be implemented in one or more embodiments.
Figure 9B illustrates cross-sectional view of the conduit structure of Figure 9A in accordance with one or more embodiments.

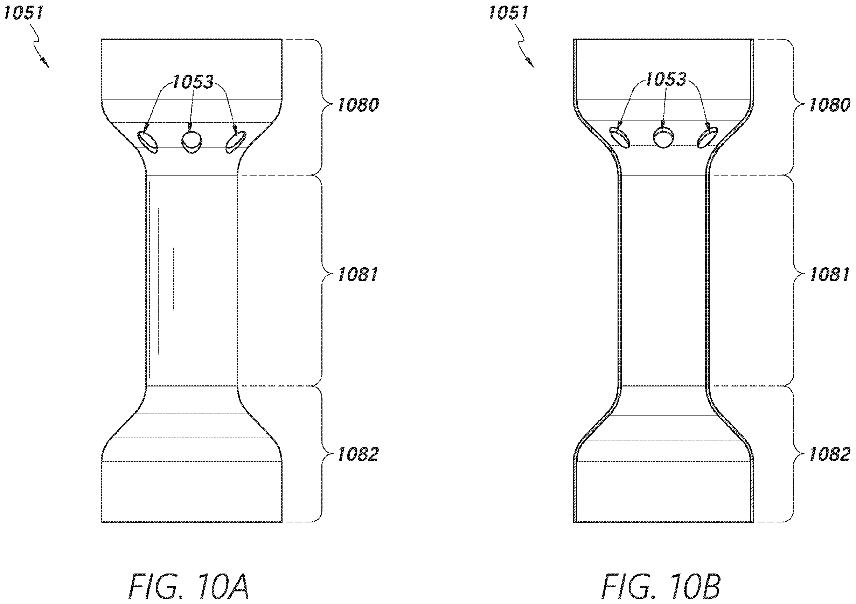

Figure 10A illustrates a side view of an example conduit structure that has a substantially hour-glass shape and that can be implemented in one or more embodiments.
Figure 10B illustrates a cross-sectional view of the conduit structure of Figure 10A in accordance with one or more embodiments.

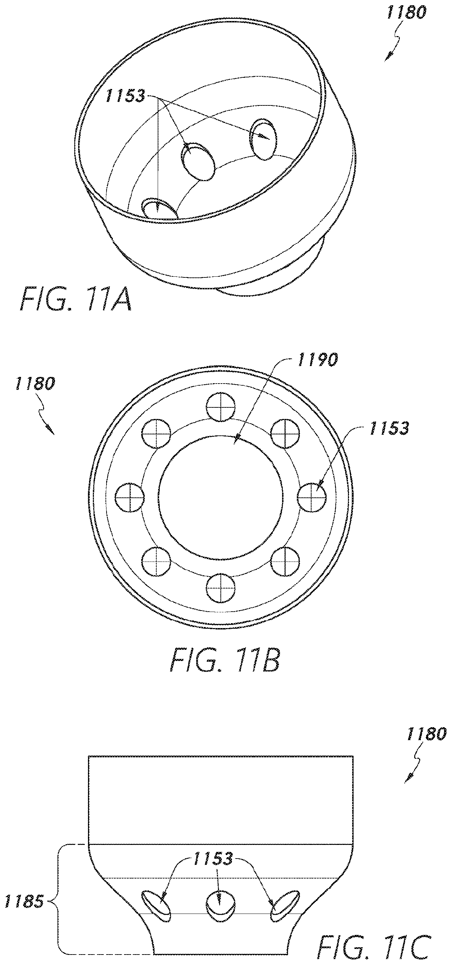

Figure 11A illustrates a perspective view of an example conduit component that includes fluid control devices and that can be implemented in one or more embodiments.
Figure 11B illustrates a top view of the conduit component of Figure 11A in accordance with one or more embodiments.
Figure 11C illustrates a side view of the conduit component of Figure 11A in accordance with one or more embodiments.

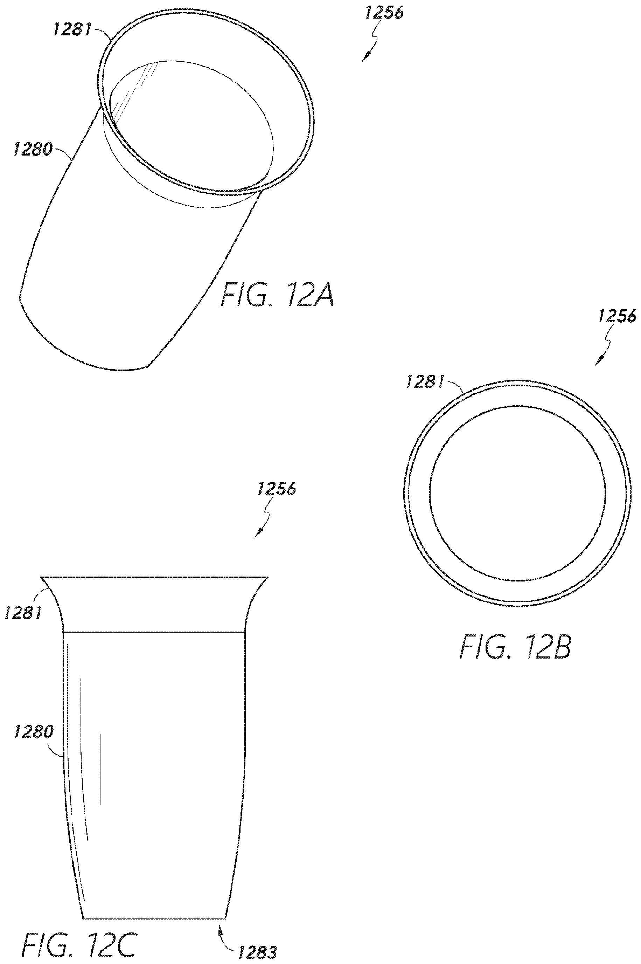

Figure 12A illustrates a perspective view of an example valve structure that has a substantially cylindrical shape and that can be implemented in one or more embodiments.
Figure 12B illustrates a top view of the valve structure of Figure 12A in accordance with one or more embodiments.
Figure 12C illustrates a side view of the valve structure of Figure 12A in accordance with one or more embodiments.

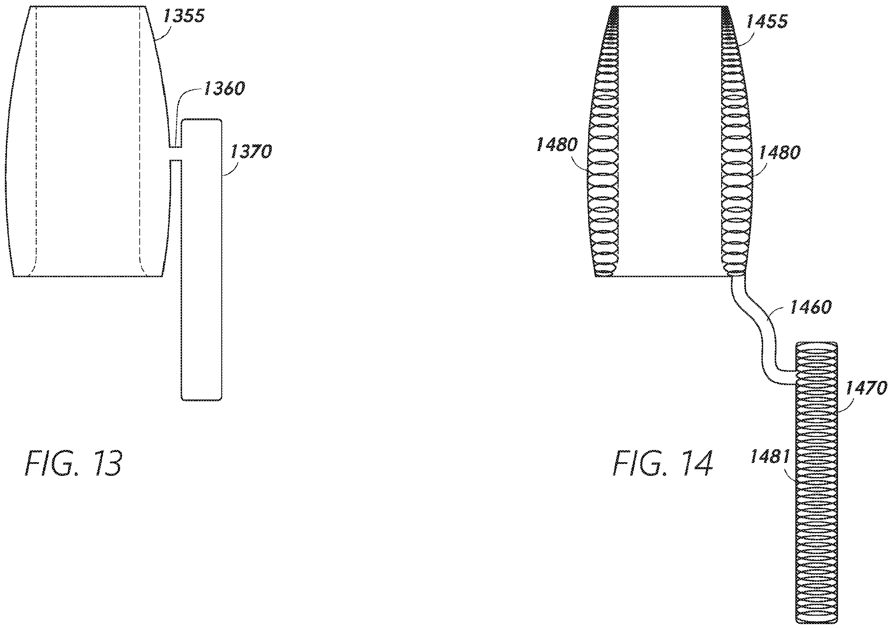

Figure 13 illustrates example expandable chamber structures that are connected at a conduit structure and that can be implemented in one or more embodiments.
Figure 14 illustrates example expandable chamber structures that include spring elements and that can be implemented in one or more embodiments.

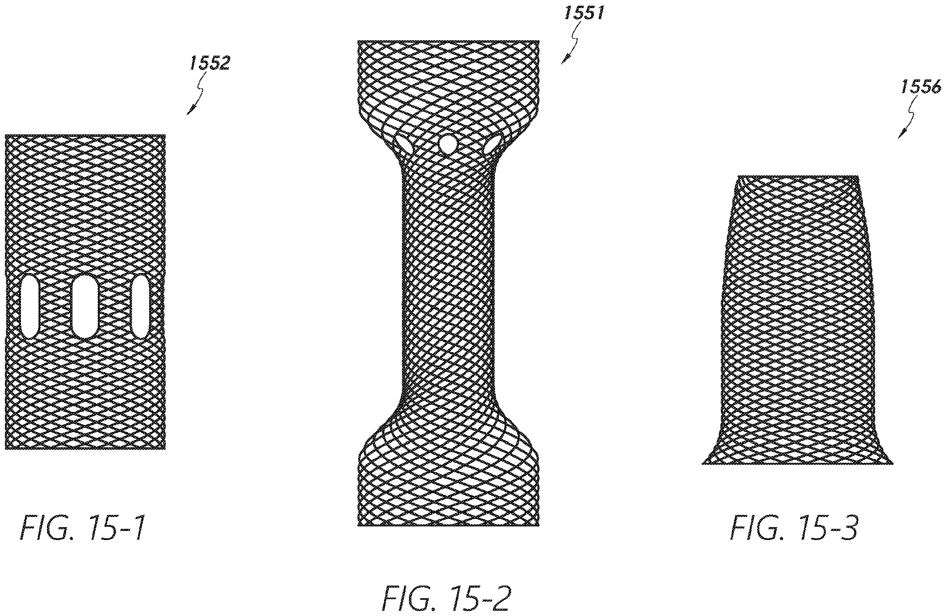

Figure 15-1 illustrates a side view of an example conduit structure that is implemented with a wire-frame structure in accordance with one or more embodiments.
Figure 15-2 illustrates a side view of another example conduit structure that is implemented with a wire-frame structure in accordance with one or more embodiments.
Figure 15-3 illustrates a side view of a valve structure that is implemented with a wire-frame structure in accordance with one or more embodiments.

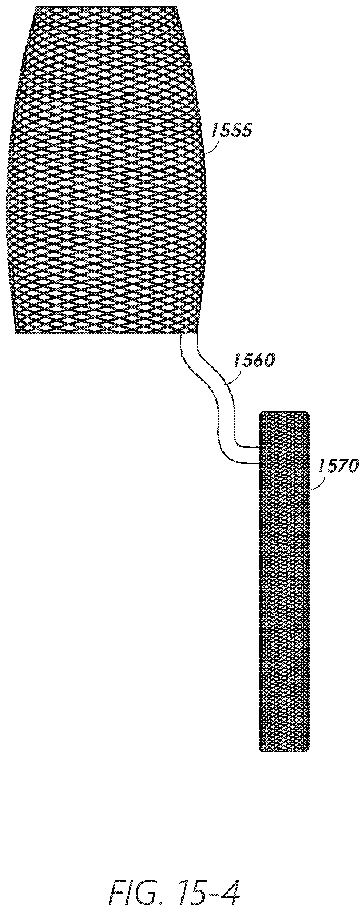

Figure 15-4 illustrates a side view of expandable chamber structures that are implemented with wire-frame structures in accordance with one or more embodiments.

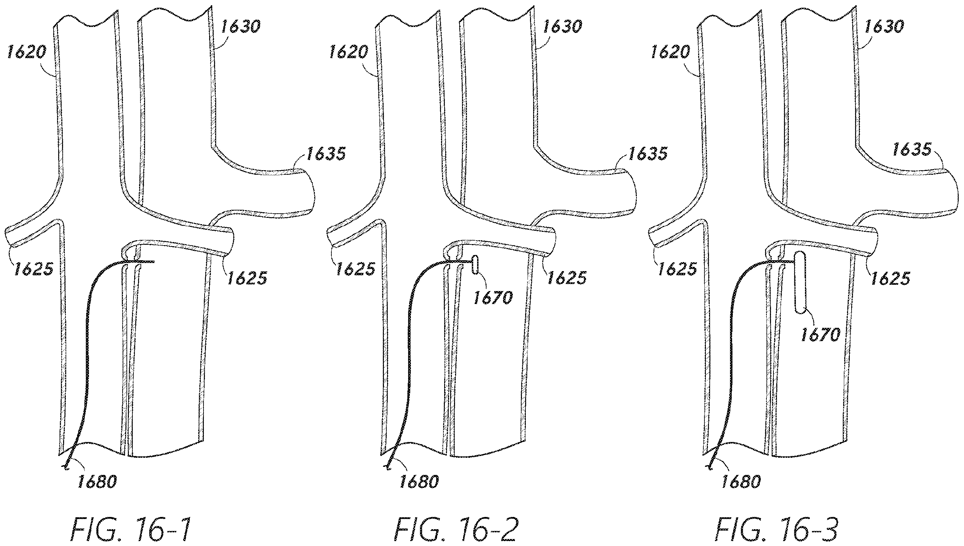

Figure 16-1 illustrates insertion of a guidewire during an example process for implanting a device in a patient in accordance with one or more embodiments.
Figure 16-2 illustrates introduction of an expandable chamber structure in a fluid vessel during the example process for implanting the device in the patient in accordance with one or more embodiments.
Figure 16-3 illustrates deployment of the expandable chamber structure during the example process for implanting the device in the patient in accordance with one or more embodiments.

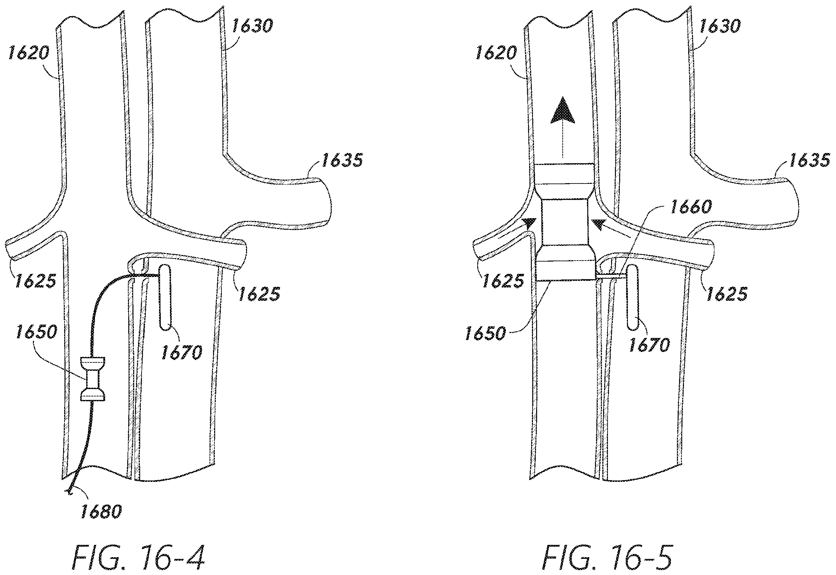

Figure 16-4 illustrates introduction of another expandable chamber structure in another fluid vessel during the example process for implanting the device in the patient in accordance with one or more embodiments.
Figure 16-5 illustrates deployment of the other expandable structure during the example process for implanting the device in the patient in accordance with one or more embodiments.

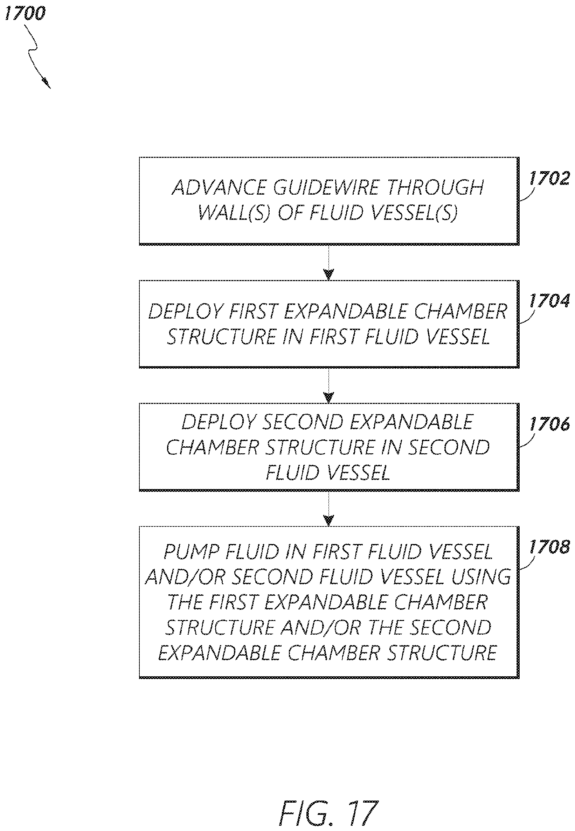

Figure 17 illustrates an example process for implanting a vessel pump within one or more vessels of a patient in accordance with one or more embodiments of the present disclosure.

### DETAILED DESCRIPTION

The headings provided herein are for convenience only and do not necessarily affect the scope or meaning of the present disclosure. The present disclosure relates to systems, devices, and methods (not claimed) to facilitate an increase in fluid flow through use of expandable chamber structures that are implantable in vessels of the human anatomy.

Although certain preferred embodiments and examples are disclosed below, inventive subject matter extends beyond the specifically disclosed embodiments and is defined by the appended claims. Thus, the scope of the claims that may arise herefrom is not limited by any of the particular embodiments described below. In any method or process disclosed herein, the acts or operations of the method or process may be performed in any suitable sequence and are not necessarily limited to any particular disclosed sequence. Various operations may be described as multiple discrete operations in turn, in a manner that may be helpful in understanding certain embodiments; however, the order of description should not be construed to imply that these operations are order dependent. Additionally, the structures, systems, and/or devices described herein may be embodied as integrated components or as separate components. For purposes of comparing various embodiments, certain aspects and advantages of these embodiments are described. Not necessarily all such aspects or advantages are achieved by any particular embodiment. Thus, for example, various embodiments may be carried out in a manner that achieves or optimizes one advantage or group of advantages as taught herein without necessarily achieving other aspects or advantages as may also be taught or suggested herein.

The term "associated with" is used herein according to its broad and ordinary meaning. For example, where a first feature, element, component, device, or member is described as being "associated with" a second feature, element, component, device, or member, such description should be understood as indicating that the first feature, element, component, device, or member is physically coupled, attached, or connected to, integrated with, embedded at least partially within, or otherwise physically related to the second feature, element, component, device, or member, whether directly or indirectly.

As noted above, congestive heart failure (CHF) is a disorder in which the heart fails to pump blood adequately to other organs in the body. In some cases, the right-side of the heart may be unable to produce sufficient suction to extract blood from the renal veins into the vena cava and return the blood to the heart through the vena cava. Since the renal veins connect to the kidneys and receive filtered blood from the kidneys, reduced blood flow in the renal veins can negatively impact the function of the kidneys. For example, the heart may be unable to return a sufficient amount of filtered blood from the kidneys to the heart, affecting kidney function. Additionally, CHF can lead to shortness of breath, exercise intolerance, fatigue, elevated venous pressure, edema, or hospitalization.

In some implementations, the present disclosure relates to devices with multiple chambers that operate in a reciprocal manner to pump fluid in a vessel. For example, the device can include a first expandable chamber structure that is implantable within a first fluid vessel, such as the abdominal aorta, and a second expandable chamber structure that is implantable within a second fluid vessel, such as the vena cava next to the renal veins. The first expandable chamber structure can be in fluid communication with the second expandable chamber structure, so that the first expandable chamber structure and the second expandable chamber structure expand and collapse in an opposing manner. For example, an increase in pressure of fluid in the first fluid vessel can cause compression of the first expandable chamber structure, resulting in expansion of the second expandable chamber structure. Further, a decrease in pressure of fluid in the first fluid vessel can cause expansion of the first expandable chamber structure, resulting in a collapse of the second expandable chamber structure. Such operation can pump fluid in the first vessel and/or the second vessel, such as pump blood to or from the heart.

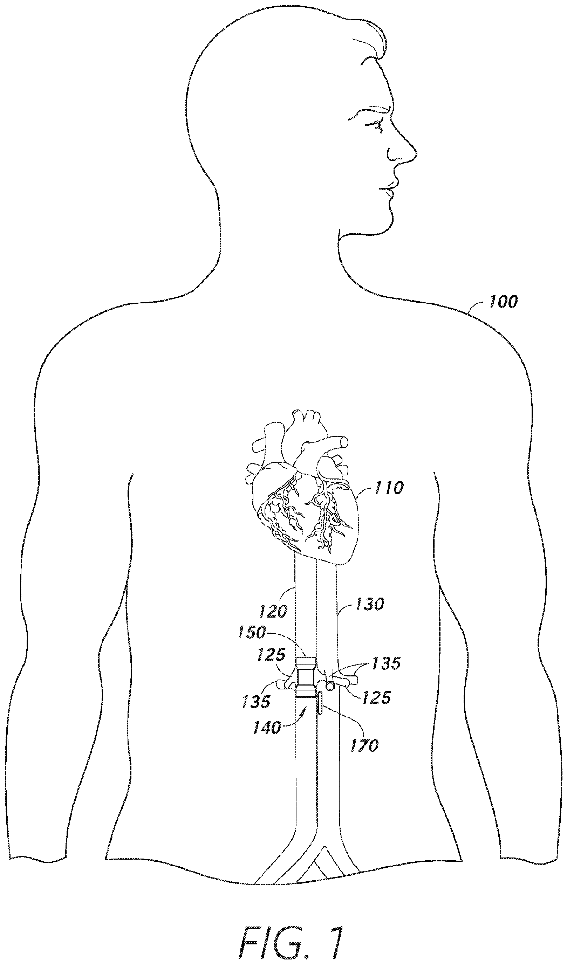

Figure 1 illustrates example human anatomy 100 having various features relevant to certain aspects of the present disclosure. The human anatomy 100 includes a heart 110 fluidly coupled to an inferior vena cava 120 and an aorta 130. In particular, the heart 110 can pump blood to various parts of the human anatomy 100 through the aorta 130 and return blood to the heart 110 through the inferior vena cava 120. For example, blood can be pumped down through the aorta 130 to arteries 135 attached to the aorta 130, such as the superior mesenteric artery, the left renal artery, and the right renal artery. Further, blood can be pulled from the renal veins 125 (i.e., the right renal vein and the left renal vein) into the inferior vena cava 120 and return to the heart 110. As illustrated, the inferior vena cava 120 is adjacent to the aorta 130. The inferior vena cava 120 and the aorta 130 represent fluid vessels. It should be understood that certain of the various vessels, organs, and/or chambers illustrated in Figure 1 are not necessarily drawn to scale, and may be represented in relatively enlarged form for the purpose of clarity in illustrating features and/or concepts that may be relevant to aspects of the present disclosure. A fluid vessel can include any anatomy that is capable of carrying fluid, such as arteries, veins, capillaries, etc. Although certain embodiments are disclosed herein with respect to blood vessels (e.g., vessels of the aortic and/or venous system(s)), it should be understood that any of the disclosed devices may be implanted at least partially within any vessel, organ, and/or chamber of a patient's anatomy, including any chamber or vessel associated with the heart and/or cardiac circulatory system(s).

In some embodiments, a device 140 (sometimes referred to herein as the "vessel pump 140") can be implanted within the inferior vena cava 120 and/or the aorta 130 to pump blood within the inferior vena cava 120 and/or the aorta 130. For example, the device 140 can include a first expandable chamber structure 170 that is implanted within the aorta 130 and an anchor structure 150 that is implanted within the inferior vena cava 120. In this illustration, the first expandable chamber structure 170 is implanted within the aorta 130 near the arteries 135 (e.g., within the abdominal aorta and within a distance to the arteries 135), while the anchor structure 150 is implanted within the inferior vena cava 120 next to the renal veins 125. The anchor structure 150 can include a second expandable chamber structure that is in fluid communication with the first expandable chamber structure 170, such as through a conduit or other structure that is implanted within walls of the inferior vena cava 120 and the aorta 130. As used herein, the term "fluid" can refer to a gas, a liquid, or a combination. For example, chamber structures disclosed herein may be expanded using any type of fluid, including saline fluid or other liquid or gas. Although illustrated as implanted near the renal veins 125, the device 140 can be implanted at other locations within the human anatomy 100, such as at other locations within the inferior vena cava 120 or the aorta 130, other fluid vessels, and so on.

In operation, the first expandable chamber structure 170 within the aorta 130 and the anchor structure 150 within the inferior vena cava 120 can operate in a cooperative manner to pump blood. For example, during systole (e.g., the heart 110 pumping blood to the body), the first expandable chamber structure 170 can collapse due to an increase in blood pressure in the aorta 130, which in turn causes the second expandable chamber structure of the anchor structure 150 to expand. Further, during diastole (e.g., the heart 110 filling with blood), the first expandable chamber structure 170 can expand due to reduced blood pressure in the aorta 130, which in turn causes the second expandable chamber structure of the anchor structure 150 to collapse. This can create suction and draw blood from the renal veins 125 into the inferior vena cava 120 to be returned to the heart 110. As such, the device 140 can actively pull blood from the renal veins 125 by the natural pulsation of the abdominal aorta. In many embodiments, the device 140 does not include a power source (e.g., a battery or other power source), but operates based on pressure/compression in the aorta 130, the inferior vena cava 120, or surrounding anatomy. Thus, the device 140 can improve renal function and/or reduce end-diastolic volume from the venous system. The reduced pressure in the inferior vena cava 120 and renal veins 125 can improve renal function and/or reduce the recurrence of systemic edema. In some embodiments, the device 140 can improve kidney functionality (e.g., improve the kidneys ability to pump out deoxygenated blood up to the inferior vena cava 120). Since kidney function and heart function are interconnected, this can bring pressure down in the inferior vena cava 120. The device 140 can also add compliance to the atrial system to reduce hypertension and/or improve atrial diastolic pressure.

The device 140 can be implemented as an inter-vessel fluid pump. A device can be considered inter-vessel when at least a component of the fluid pump is disposed/implanted within each of a plurality of vessels or portions of a continuous and/or contiguous portions of a single vessel. Although many examples are discussed herein in the context of the first expandable chamber structure 170 in the aorta 130 causing blood to be pumped within the inferior vena cava 120, the device 140 can operate in other manners, such as the anchor structure 150 in the inferior vena cava 120 causing blood to be pumped within the aorta 130. In some implementations, multiple expandable chamber structures are implanted in, and produce increased blood flow in, either the aortic system or the venous system, but not both.

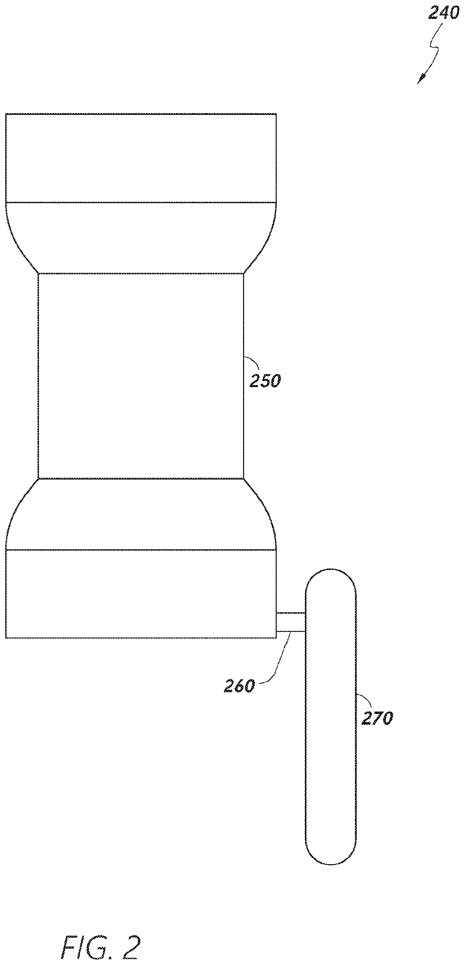

Figure 2 illustrates an example device 240 that includes a first expandable chamber structure 270 and an anchor structure 250 to pump a fluid in accordance with one or more embodiments of the present disclosure. The device 240 includes the first expandable chamber structure 270, the anchor structure 250, and a conduit structure 260 to connect the first expandable chamber structure 270 and the anchor structure 250 with a fluid. The first expandable chamber structure 270 can be implanted within a first fluid vessel, such as the aorta 130 of Figure 1, and the anchor structure 250 can be implanted within a second fluid vessel, such as the inferior vena cava 120 of Figure 1. The anchor structure 250 can include a second expandable chamber structure that is connected to the first expandable chamber structure 270 through the conduit structure 260. In some embodiments, the conduit structure 260 can be rigid or semi-rigid to prevent expansion of the conduit structure 260. This may avoid the conduit structure 260 from expanding and damaging a wall of a fluid vessel where the conduit structure 260 is implanted, such as a wall of an aorta or inferior vena cava. Although the conduit structure 260 can be formed from any material whether rigid or flexible.

Although the device 240 is discussed with two expandable chamber structures, any number of expandable chamber structures can be implemented. For example, a first expandable chamber structure can be implemented within a first fluid vessel, a second expandable chamber structure can be implemented within a second fluid vessel, and third expandable chamber structure can be implemented within a third fluid vessel. Here, expansion and contraction of the first expandable chamber structure can cause contraction and expansion of the second expandable chamber structure and the third expandable chamber structure in a unified manner so that fluid is simultaneously pumped within the second fluid vessel and the third fluid vessel.

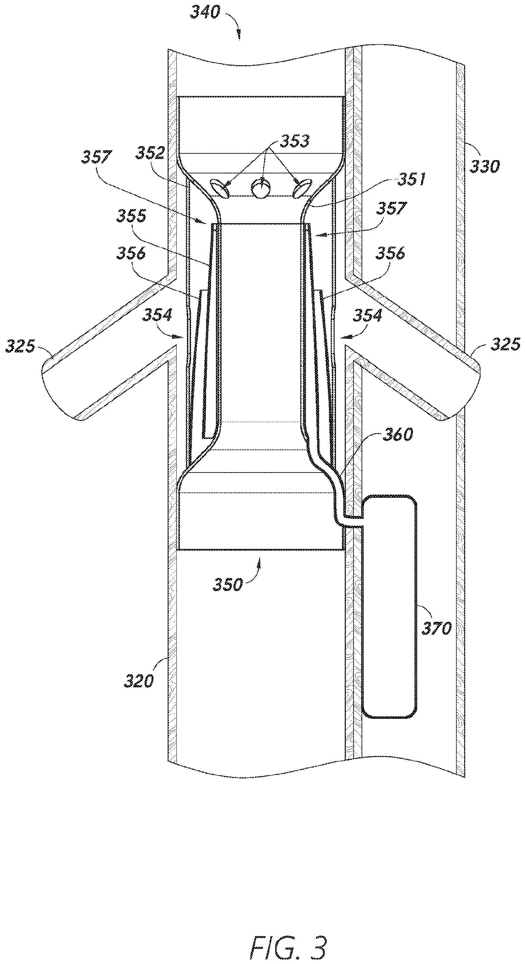

Figure 3 illustrates a cross-sectional view of an aorta 330 and an inferior vena cava 320 with an example inter-vessel fluid pump device/assembly 340 implanted therein to pump blood and/or increase blood flow within one or more of the illustrated blood vessels and/or portions thereof in accordance with one or more embodiments of the present disclosure. As illustrated, the device 340 includes a first at least partially expandable chamber structure 370 connected to an anchor structure 350 via a conduit structure 360. The anchor structure 350 is implanted within the inferior vena cava 320 and is associated with a second expandable chamber structure 355. The first expandable chamber structure 370 is advantageously in fluid communication with the second expandable chamber structure 355 through the conduit structure 360, which extends through a wall of the aorta 330 and a wall of the inferior vena cava 320. As used herein, the term "expandable chamber structure" can generally refer to a structure and a cavity within the structure. For example, a chamber structure can include one or more walls that form an at least partially fluid-tight cavity. Further, the term "conduit structure" can generally refer to a structure and a cavity within the structure. For example, a conduit structure can comprise a balloon, tube, hose, or other structure forming a lumen therein to enable a fluid to pass through and/or be contained therein.

The anchor structure 350 also includes a first conduit structure 351, a second conduit structure 352, and a valve structure 356. The first conduit structure 351 can have a substantially hour-glass-shaped profile and a lumen that extends longitudinally through a center of the first conduit structure 351. As illustrated, the first conduit structure 351 can also include fluid control devices 353 to permit or block fluid flow from a suction cavity 357. The fluid control devices 353 can include one-way valves that permit fluid flow in one direction, holes, or other structures. The second conduit structure 352 can have a substantially cylindrical shape and one or more holes 354 that extend radially. The second conduit structure 352 can be configured to house at least a portion of the first conduit structure 351. The valve structure 356 is configured to align longitudinally with the first conduit structure 351 and can be disposed between the second expandable chamber structure 355 and the second conduit structure 352. The valve structure 356 can be configured to be displaced in a radial direction (e.g., towards the second conduit structure 352) to block fluid flow through the holes 354 of the second conduit structure 352, as discussed in further detail below. Although the first conduit structure 351, the second conduit structure 352, and the valve structure 356 are illustrated with various cross-sectional-shapes, such components can include other forms, such as hyper-rectangular forms, elliptical-forms, and so on. Further, although many embodiments are illustrated with a single expandable chamber structure in the first expandable chamber structure 270 and a single expandable chamber structure in the anchor structure 250, any number of expandable chamber structures can be implemented.

As shown, the components of the anchor structure 350 are disposed to create the suction cavity 357. In particular, the second conduit structure 352 is disposed around the first conduit structure 351, so that a spacing exists between the first conduit structure 351 and the second conduit structure 352. The second expandable chamber structure 355 is disposed between the first conduit structure 351 and the second conduit structure 352 and expands to fill at least a portion of the suction cavity 357 illustrated in Figure 3. As illustrated in Figure 3, the second expandable chamber structure 355 can sit on a bottom portion of the first conduit structure 351, such as a portion of the first conduit structure 351 where a diameter of the first conduit structure 351 increases. The valve structure 356 is disposed between the second expandable chamber structure 355 and the second conduit structure 352. In such a configuration, the components create the suction cavity 357, which extends circumferentially around the first conduit structure 351.

In some embodiments, when in an implanted state, such as that shown in Figure 3, an upper portion of the first conduit structure 351 (having a larger diameter) contacts or is placed in proximity to an interior surface of the inferior vena cava 320 and a lower portion of the first conduit structure 351 (having the larger diameter) contacts or is placed in proximity to the interior surface of the inferior vena cava 320. The upper portion of the first conduit structure 351 can be placed above renal veins 325 and the lower portion of the first conduit structure 351 can be placed below the renal veins 325. This results in a central portion of the first conduit structure 351 (having a smaller diameter) being positioned near the renal veins 325. In some embodiments, one or more anchors are attached to the inferior vena cava 320 (above and/or below the anchor structure 350) and attached to the anchor structure 350 to maintain the anchor structure 350 in a substantially fixed position. Similarly, one or more anchors are attached to the aorta 330 (above and/or below the first expandable chamber structure 370) and attached to the first expandable chamber structure 370 to maintain the first expandable chamber structure 370 in a substantially fixed position.

Although not illustrated in Figure 3, in some embodiments, the device 340 can include a port to connect to a control device that is configured to control one or more characteristics of the device 340. For example, the first expandable chamber structure 370 and/or the second expandable chamber structure 355 can be connected to a control device (which can include processing circuitry and/or memory) that is located externally to a human, such as on the skin or within a layer of the skin. The control device can regulate pressure or fluid in the first expandable chamber structure 370 and/or the second expandable chamber structure 355, such as an amount of fluid within the expandable chamber structures 370 and 355. By doing so, the control device can regulate an amount of blood flow that the device 340 is able to pump, a pressure needed to initiate pumping of blood, or various other characteristics of the device 340. In some embodiments, the control device can enable a fluid within the first expandable chamber structure 370 and/or the second expandable chamber structure 355 to be replaced. The control device can include an external injection port to facilitate such replacement, in some instances.

Figure 4-1 through 4-6 illustrate components of an example device that is configured to pump fluid in accordance with one or more embodiments of the present disclosure.

Figure 4-1 illustrates an example upper portion 451A of a first conduit structure 451. The upper portion 451A can include fluid control devices 453, such as one-way valves, holes, or other structures. An end 461 of the upper portion 451A can be configured to connect to a lower portion 451B of the first conduit structure 451 illustrated in Figure 4-2. The upper portion 451A can be connected to the lower portion 451B in a variety of manners, such as through threading, an adhesive, or other attachment mechanism. As such, the upper portion 451A and/or the lower portion 451B can include threading or other attachment mechanisms.

Figure 4-2 illustrates the lower portion 451B of the first conduit structure 451. An end 463 of the lower portion 451B can be configured to connect to the upper portion 451A of the first conduit structure 451 through a variety of mechanisms, as discussed above. Although the upper portion 451A and the lower portion 451B are illustrated as separate components, in some embodiments the upper portion 451A and the lower portion 451B form a singular unitary form or are connected at other locations.

Figure 4-3 illustrates an example second conduit structure 452 that is configured to associate with the first conduit structure 451. As illustrated, the second conduit structure 452 has a substantially cylindrical shape and includes holes 454 around a circumference of the second conduit structure 452. The holes 454 extend radially through the second conduit structure 452. Although a particular number of the holes 454 are illustrated, any number of holes can be implemented.

Figure 4-4 illustrates an example second expandable chamber structure 455 that is configured to be implemented with the first conduit structure 451 and the second conduit structure 452. Here, the second expandable chamber structure 455 is shown in a substantially collapsed state. In the collapsed state, the second expandable chamber structure 455 can include a substantially cylindrical form with a lip 462 extending radially off a main body of the second expandable chamber structure 455.

Figure 4-5 illustrates an example valve structure 456 that is configured to be implemented with the first conduit structure 451, the second conduit structure 452, and the second expandable chamber structure 455 to block or permit fluid flow through the holes 454. The valve structure 456 can include a substantially cylindrical form and a lip 464 that extends radially from a main body of the valve structure 456. In some embodiments, the valve structure 456 can have a diameter that is larger than a diameter of the second expandable chamber structure 455 in a collapsed state. This can allow the valve structure 456 to be disposed around the second expandable chamber structure 455.

Figure 4-6 illustrates an example first expandable chamber structure 470 that is configured to be implemented with the second expandable chamber structure 455. The first expandable chamber structure 470 can include a substantially cylindrical form, as shown. The first expandable chamber structure 470 can include a structure that encloses a fluid. The first expandable chamber structure 470 can include a first end 465 and a second end 466. Although not illustrated, the first expandable chamber structure 470 can be connected to the second expandable chamber structure 455 through a conduit or other mechanism. Further, in some embodiments the first expandable chamber structure 470 and the second expandable chamber structure 455 form a unitary form.

Although the upper portion 451A, the lower portion 451B, the second conduit structure 452, the second expandable chamber structure 455, the valve structure 456, and the first expandable chamber structure 470 are illustrated with substantially cylindrical forms, such components can include a variety of forms (e.g., in an expanded/collapsed state). For example, any of such components can be implemented in a hyper-rectangular form, elliptical form, or another form.

Figure 5-1 through 5-3 illustrate aspects of an example diastole phase of a cardiac cycle for a device 540 that is configured to pump fluid in accordance with one or more embodiments of the present disclosure. Figures 5-1 and 5-2 illustrate a state of the device 540 when aortic pressure is at or near a minimum, and Figure 5-3 illustrates a graph 580 of aortic pressure in mmHg over time in seconds. A point 582 on the graph 580 illustrates a time when aortic pressure is at a minimum.

As illustrated in Figures 5-1 and 5-2, the device 540 includes a first expandable chamber structure 570 and a second expandable chamber structure 555 that is in fluid communication with the first expandable chamber structure 570 through a conduit structure 560. When implanted, as illustrated in Figure 5-2, the first expandable chamber structure 570 is positioned in an aorta 530 and the second expandable chamber structure 555 is positioned within an inferior vena cava 520, with the conduit structure 560 extending through a wall of the aorta 530 and a wall of the inferior vena cava 520. The device 540 also includes a first conduit structure 551, a second conduit structure 552, a valve structure 556, and a suction cavity 557 between the first conduit structure 551 and the second conduit structure 552. In some embodiments, when in an implanted state, an upper portion of the first conduit structure 551 (having a larger diameter) contacts or is placed in proximity to an interior surface of the inferior vena cava 520, a central portion of the first conduit structure 551 (having a smaller diameter) is positioned around renal veins 525, and a lower portion of the first conduit structure 551 (having the larger diameter) contacts or is placed in proximity to the interior surface of the inferior vena cava 520. In such position, the device 540 can seal against the interior surface of the inferior vena cava 520 to create a suction zone around the renal veins 525, which facilitates blood being drawn from the renal veins 525, as discussed below. This suction zone can be created at least in part from the hour-glass shape of the device 540.

In some embodiments, the device 540 can operate based on a change in aortic blood pressure (e.g., a rise or fall of pressure in the aorta 530). For example, during diastole, aortic blood pressure decreases (as illustrated in Figure 5-3) and the heart (not illustrated) refills with blood by pulling blood through the inferior vena cava 520 and other vessels. As aortic blood pressure decreases, the first expandable chamber structure 570 expands, causing the second expandable chamber structure 555 to collapse and the valve structure 556 to be displaced radially inward away from holes 554 in the second conduit structure 552 (e.g., opening the holes 554 for fluid flow). This creates suction in the suction cavity 557, which causes fluid control devices 553 in the first conduit structure 551 (which can be implemented as one-way valves) to close and block blood in the inferior vena cava 520 from flowing down into the suction cavity 557. This suction also pulls blood from the renal veins 525 through the holes 554 and into the suction cavity 557. Figures 5-1 and 5-2 illustrate a state of the device 540 during diastole when the first expandable chamber structure 570 is expanded and the second expandable chamber structure 555 is collapsed (e.g., at a time when aortic pressure is at or near a minimum). As illustrated in Figure 5-2, during diastole, blood can also flow in a normal manner through the inferior vena cava 520 by flowing through an interior portion of the device 540 (e.g., a lumen through the center of the first conduit structure 551). The dark arrows in Figure 5-2 show blood flow during diastole (e.g., blood flow from the renal veins 525 into the suction cavity 557 and blood flow through a center lumen in the first conduit structure 551).

In some embodiments, blood that is collected from the renal veins 525 into the suction cavity 557 during diastole is pumped out of the suction cavity 557 (through the fluid control devices 553 in the first conduit structure 551) during systole, as discussed below. However, in some embodiments, blood that is collected in the suction cavity 557 can be at least partially pumped out of the suction cavity 557 (through the fluid control devices 553) during diastole.

Figure 6-1 through 6-3 illustrate aspects of an example systole phase of a cardiac cycle for a device 640 that is configured to pump fluid in accordance with one or more embodiments of the present disclosure. Figures 6-1 and 6-2 illustrate a state of the device 640 when aortic pressure is at or near a maximum, and Figure 6-3 illustrates a graph 680 of aortic pressure in mmHg over time in seconds. A point 682 on the graph 680 illustrates a time when aortic pressure is at a maximum.

As illustrated in Figures 6-1 and 6-2, the device 640 includes a first expandable chamber structure 670 and a second expandable chamber structure 655 that is in fluid communication with the first expandable chamber structure 670 through a conduit structure 660. When implanted, as illustrated in Figure 6-2, the first expandable chamber structure 670 is positioned in an aorta 630 and the second expandable chamber structure 655 is positioned within an inferior vena cava 620, with the conduit structure 660 extending through a wall of the aorta 630 and a wall of the inferior vena cava 620. The device 640 also includes a first conduit structure 651, a second conduit structure 652, a valve structure 656, and a suction cavity 657 between the first conduit structure 651 and the second conduit structure 652. In some embodiments, when in an implanted state, an upper portion of the first conduit structure 651 (having a larger diameter) contacts or is placed in proximity to an interior surface of the inferior vena cava 620, a central portion of the first conduit structure 651 (having a smaller diameter) is positioned around renal veins 625, and a lower portion of the first conduit structure 651 (having the larger diameter) contacts or is placed in proximity to the interior surface of the inferior vena cava 620. In such position, the device 640 can create a suction zone around the renal veins 625 to draw blood from the renal veins 625, as discussed above in reference to Figures 5-1 through 5-3.

In some embodiments, the device 640 can operate based on a change in aortic blood pressure (e.g., a rise or fall of pressure in the aorta 630). For example, during systole, aortic blood pressure increases (as illustrated in Figure 6-3) and the heart (not illustrated) pumps blood through the aorta 630 and other vessels. As aortic blood pressure increases, the first expandable chamber structure 670 collapses (e.g., is compressed), causing the second expandable chamber structure 655 to expand radially and the valve structure 656 to be displaced radially outward to block holes 654 in the second conduit structure 652. As such, the valve structure 655 can block blood flow from the renal veins 625 into the suction cavity 657. Further, as the second expandable chamber structure 655 expands, pressure in the suction cavity 652 increases, causing fluid control devices 653 to open and blood in the suction cavity 657 to be pushed out of the suction cavity 657 through the fluid control devices 653. In some embodiments, the fluid control devices 653 are implemented as one-way valves that allow blood flow from the suction cavity 657 into the inferior vena cava 620 (e.g., when the pressure is above an amount in the suction cavity 657) and block blood flow from the inferior vena cava 620 into the suction cavity 657. Figures 6-1 and 6-2 illustrate a state of the device 640 during systole when the first expandable chamber structure 670 is collapsed and the second expandable chamber structure 655 is expanded (e.g., at a time when aortic pressure is at or near a maximum). The dark arrows in Figure 6-2 show blood flow during systole (e.g., blood flow from the suction cavity 657 into an interior portion of the first conduit structure 651 and the inferior vena cava 620).

Although a valve structure is illustrated in Figures 5-2 and 6-2, in some embodiments a valve structure is not implemented and an expandable chamber structure functions to permit or block fluid flow into a suction cavity. For example, the device 640 can be implemented without the valve structure 656. Here the second expandable chamber structure 655 can expand and collapse to block and open the holes 654 (e.g., block or allow blood flow into the suction cavity 657).

Figures 7A-7B illustrate example expandable chamber structures that can be implemented in one or more embodiments of the present disclosure. In particular, Figure 7A shows a side view of a first expandable chamber structure 770 connected to a second expandable chamber structure 755 via a conduit structure 760. Figure 7B shows a top view of the first expandable chamber structure 770 and the second expandable chamber structure 755 of Figure 7A. Although the conduit structure 760 is illustrated in Figures 7A and 7B, in some embodiments the conduit structure 760 is not implemented and the first expandable chamber structure 770 is connected directly to the second expandable chamber structure 755.

The second expandable chamber structure 755 can include an interior surface 780 that is substantially cylindrical in shape. A diameter of the interior surface 780 can be designed to a diameter of a conduit structure over which the second expandable chamber structure 755 can be placed. In some embodiments where the conduit structure has an hour-glass shape, a lower portion 782 of the interior surface 780 can extend radially outward to sit on a lower portion of the conduit structure, which can have a slightly larger diameter. In some embodiments, the second expandable chamber structure 755 has a tapered cylindrical shape. For example, outer surface of an upper portion of the second expandable chamber structure 755 can have a smaller diameter than an outer surface of the middle or lower portion of the second expandable chamber structure 755 when in an expanded or collapsed state.

The first expandable chamber structure 770, the second expandable chamber structure 755, and/or the conduit structure 760 can be filled with fluid. For example, the first expandable chamber structure 770, the second expandable chamber structure 755, and/or the conduit structure 760 can form a closed system that is filled with saline, air, etc. In some embodiments, the first expandable chamber structure 770, the second expandable chamber structure 755, and/or the conduit structure 760 are filled to a particular pressure (e.g., are pressurized). The first expandable chamber structure 770 and the second expandable chamber structure 755 can generally exchange fluid freely based on a pressure exerted on the first expandable chamber structure 770 or the second expandable chamber structure 755. For example, if a pressure is exerted on an outer surface of the first expandable chamber structure 770, this can cause fluid in the first expandable chamber structure 770 to be transferred to the second expandable chamber structure 755. In some embodiments, the first expandable chamber structure 770 (and/or the second expandable chamber structure 755) can be associated with hemodynamic performance similar to an intra-aortic balloon pump (IABP).

The first expandable chamber structure 770, the second expandable chamber structure 755, and/or the conduit structure 760 can be formed of a structure and/or material that is configured to expand or contract. In some embodiments, the first expandable chamber structure 770, the second expandable chamber structure 755, and/or the conduit structure 760 can be formed of a flexible mesh or wire-frame structure that generally maintains a particular form unless sufficient force is applied. The mesh or wire-frame structure can be formed of metal, plastic, or other material, and/or can include a material disposed therein, such as silicone, plastic, or the like. Further, in some embodiments, the first expandable chamber structure 770 and/or the second expandable chamber structure 755 (or the conduit structure 760) can be implemented as a compliant or semi-compliant balloon. As described herein, the term "compliant" or "compliance" can refer to the ability of an item to distend and increase in volume with increasing pressure, or the tendency of an item to resist recoil toward its original dimensions on application of a distending or compressing force. For example, compliance of an expandable chamber structure can refer to the ability of the expandable chamber structure to stretch in response to an applied pressure. In some embodiments, an expandable chamber structure can have more or less compliance than another expandable chamber structure. For example, the first expandable chamber structure 770 and the second expandable chamber structure 755 can each be implemented as a balloon, wherein the second expandable chamber structure 755 is more compliant (e.g., has more stretch) than the first expandable chamber structure 770. However, in other examples the compliance of the first expandable chamber structure 770 and the second expandable chamber structure 755 can be switched. In yet other embodiments, the first expandable chamber structure 770 and/or the second expandable chamber structure 755 (or the conduit structure 760) can be implemented as non-compliant balloons. As such, the first expandable chamber structure 770 and/or the second expandable chamber structure 755 (or the conduit structure 760) can have particular compliance characteristics. The first expandable chamber structure 770, the second expandable chamber structure 755, and/or the conduit structure 760 can be formed of various materials, such as polyurethane, silicone, metal, plastic, and so on.

In some embodiments, the first expandable chamber structure 770 and/or the second expandable chamber structure 755 can be designed to expand and/or collapse over a specific pressure range. For example, the first expandable chamber structure 770 can be configured to be collapse when a pressure (within a range of aortic pressure associated with a systole phase of a cardiac cycle) is applied to the first expandable chamber structure 770, and can be configured to expand when a pressure (within a range of aortic pressure associated with a diastole phase of a cardiac cycle) is applied to the first expandable chamber structure 770. Alternatively or additionally, the second expandable chamber structure 755 can be configured to be expand in response to an applied pressure within a specific range and collapse in response to an applied pressure within another range.

Figures 8A-8B illustrate an example anchor structure 850 that includes conduit structures in an attached configuration and that can be implemented in one or more embodiments of the present disclosure. In particular, Figure 8A shows a side view of the anchor structure 850, while Figure 8B shows a cross-sectional view of the anchor structure 850. Here, the anchor structure 850 includes a first conduit structure 851 attached to a second conduit structure 852. For example, the second conduit structure 852 is disposed around a central portion of the first conduit structure 851. Although discussed as separate components in many embodiments, the first conduit structure 851 and the second conduit structure 852 can be a single unified component. The first conduit structure 851 can include fluid control devices 853 disposed in an upper portion of the first conduit structure 851, such as a section between a smallest diameter of the first conduit structure 851 and a largest diameter of the first conduit structure 851. Although the fluid control devices 853 are illustrated at an upper location in Figure 8B, the fluid control devices 853 can be located at other locations, such as in a central portion 880 of the first conduit structure 851 or a lower portion of the first conduit structure 851. The second conduit structure 852 can include holes 854 disposed in a central portion of the second conduit structure 852. Although holes are discussed, in some embodiments the holes 854 can be replaced with one-way valves or other structures. The first conduit structure 851 and the second conduit structure 852 can be attached to create a cavity 857. In some embodiments, the second conduit structure 852 can contact the first conduit structure 851 at upper and lower portions of the first conduit structure 851 so that a fluid is only able to enter or leave the cavity 857 through the fluid control devices 853 and the holes 854. Although a particular number of the holes 854 and the fluid control devices 853 are illustrated in Figures 8A and 8B, any number of holes and or fluid control devices 853 can be implemented.

Figures 9A-9B illustrate an example conduit structure 952 that has a substantially cylindrical form and that can be implemented in one or more embodiments of the present disclosure. In particular, Figure 9A shows a side view of the conduit structure 952, while Figure 9B shows a cross-sectional view of the conduit structure 952. The conduit structure 952 can include holes 954 disposed circumferentially around the conduit structure 952. In some embodiments, such as that illustrated in Figures 9A and 9B, the holes 954 are disposed around a central portion of the conduit structure 952. In other embodiments, the holes 954 can be disposed at other locations on the conduit structure 952. The conduit structure 952 can include a cylinder, with a wall of the cylinder having a particular thickness. However, in other embodiments the conduit structure 952 can have other forms, such as a hyper-rectangular form or any other form. In some embodiments, the conduit structure 952 can be configured to have a rigid or semi-rigid structure.

Figures 10A-10B illustrate an example conduit structure 1051 that has a substantially hour-glass shape and that can be implemented in one or more embodiments of the present disclosure. In particular, Figure 10A shows a side view of the conduit structure 1051, while Figure 10B shows a cross-sectional view of the conduit structure 1051. The conduit structure 1051 can include an upper portion 1080, a central portion 1081, and a lower portion 1082. As illustrated, the upper portion 1080 increases in diameter (in a direction toward an outer end of the upper portion 1080) from a diameter of the central portion 1081 to a largest diameter of the upper portion 1080. Similarly, the lower portion 1082 decreases in diameter (in a direction toward an outer end of the lower portion 1082) from a diameter of the central portion 1081 to a largest diameter of the lower portion 1022. A largest diameter of the upper portion 1080 and a largest diameter of the lower portion 1082 can each be larger than a diameter of the central portion 1081. Further, a diameter of the central portion 1081 can be smaller than a diameter of another conduit structure, such as the conduit structure 952 of Figures 9A and 9B, that is configured to be disposed around the conduit structure 1051. The upper portion 1080 can include fluid control devices 1053, such as one-way valves, holes, etc. The conduit structure 1051 can be formed as a single unitary component or various components. A wall of the conduit structure 1051 can have a variety of thicknesses. As illustrated, an interior portion of the conduit structure 1051 can comprise a lumen (e.g., an open passage) that extends longitudinally through the conduit structure 1051 to allow fluid to flow in either direction axially through the conduit structure 1051. In some embodiments, the conduit structure 1051 can be configured to have a rigid or semi-rigid structure when deployed.

Figures 11A-11C illustrate an example conduit component 1180 that includes fluid control devices 1183 and that can be implemented in one or more embodiments of the present disclosure. In particular, Figure 11A shows a perspective view of the conduit component 1180, Figure 11B shows a top view of the conduit component 1180, and Figure 11C shows a side view of the conduit component 1180. In some embodiments, the conduit component 1180 is implemented as a portion of an hour-glass conduit structure, such as the hour-glass conduit structure 1051 of Figures 10A and 10B. For example, the conduit component 1180 can be attached to the central portion 1081 of the hour-glass conduit structure 1051 of Figures 10A and 10B. As illustrated in Figures 11A-11C, the conduit component 1180 includes the fluid control devices 1153 disposed circumferentially in a portion 1085 where a diameter of the conduit component 1180 changes. The fluid control devices 1153 can include one-way valves, holes, or other devices. For ease of illustration, Figures 11A and 11C (and in various other Figures) illustrate the fluid control devices 1153 as openings, while Figure 11B illustrates the fluid control devices 1153 as one-way valves. A center portion 1190 of the conduit component 1180 can generally be open to allow fluid to flow axially through the conduit component 1180. However, in some embodiments the center portion 1190 can include a one-way valve or other component.

Figures 12A-12C illustrate an example valve structure 1256 that has a substantially cylindrical shape and that can be implemented in one or more embodiments of the present disclosure. In particular, Figure 12A shows a perspective view of the valve structure 1256, Figure 12B shows a top view of the valve structure 1256, and Figure 12C shows a side view of the valve structure 1256. In some embodiments, the valve structure 1256 can be referred to as a renal veins valve, since the valve structure 1256 can function, when deployed, to control blood flow from renal veins into a suction chamber of a device that operates to pump blood. As illustrated, the valve structure 1256 can include a first portion 1280 and a second portion 1281. The second portion 1281 can extend radially so that a distal end of the second portion 1281 with respect to the first portion 1280 has a larger diameter than the first portion 1280. The second portion 1281 can be configured to sit on a lower portion of a conduit structure, such as the lower portion 1082 of the conduit structure 1051 of Figures 10A and 10B. The valve structure 1256 can be formed of a flexible material such as plastic, silicone, or another material, so that the valve structure 1256 can expand (or be displaced) radially. In Figures 12A-12C, the valve structure 1256 includes a tapered end 1283 (e.g., a portion where a diameter decreases). However, in other embodiments the valve structure 1256 may not include a tapered portion.

Figure 13 illustrates example expandable chamber structures 1370 and 1355 that are connected via a conduit structure 1360 and that can be implemented in one or more embodiments of the present disclosure. In particular, the first expandable chamber structure 1370 is connected to the second expandable chamber structure 1355 at substantially a central portion of the second expandable chamber structure 1355. Here, the conduit structure 1360 is relatively short in length (e.g., less than a distance) to illustrate that the first expandable chamber structure 1370 and the second expandable chamber structure 1355 can be connected in almost a direct manner without a channel between the first expandable chamber structure 1370 and the second expandable chamber structure 1355. In some embodiments, the conduit structure 1360 can be removed entirely and the first expandable chamber structure 1370 can be connected to the second expandable chamber structure 1355 to form an almost unitary expandable chamber structure.

Figure 14 illustrates example expandable chamber structures 1470 and 1455 that include spring elements 1481 and 1480, respectively, and that can be implemented in one or more embodiments of the present disclosure. The spring elements 1481 and 1480 can be disposed within the expandable chamber structures 1470 and 1455, respectively. The spring elements 1481 and 1480 can serve as chamber support structure that supports the expandable chamber structures 1470 and 1455 (which can be implemented with balloons), wherein the chamber support structure can be configured to exert outward force on an inner surface of an expandable chamber structure to resist collapsing and/or assist with chamber expansion rebound. In some embodiments, the spring elements 1481 and/or 1480 can include a metal or plastic frame that is configured to maintain a particular form unless a sufficient force is applied thereto. Although the first expandable chamber structure 1470 and the second expandable chamber structure 1455 are each illustrated in Figure 14 as including a spring element, in some embodiments only one of the expandable chamber structures is associated with a spring element.

Figures 15-1 through 15-4 illustrate device components that are implemented with wire-frame structures in accordance with one or more embodiments of the present disclosure. In particular, Figure 15-1 illustrates a side view of a second conduit structure 1552 that is configured to be associated with a first conduit structure 1551, Figure 15-2 illustrates a side view of the first conduit structure 1551 that has a substantially hour-glass shape, Figure 15-3 illustrates a side view of a valve structure 1556, and Figure 15-4 illustrates a side view of expandable chamber structures 1570 and 1555 connected via a conduit structure 1560. In some embodiments, a wire-frame structure includes a partially rigid frame, which can comprise an expandable metal or plastic frame covered and/or filled by a sleeve or covering, such as silicone, plastic, or another flexible material. In some embodiments, the second conduit structure 1552, the first conduit structure 1551, the valve structure 1556, and/or the expandable chamber structures 1570 and 1555 (or any other component discussed herein) can be implemented as a stent (sometimes referred to as a "stent structure"), such as a stent similar to an esophageal stent. A stent can include a wire-frame structure. In a compressed or collapsed configuration, a stent can be transportable to a target implantation location using a catheter, as discussed in further detail below in reference to Figure 16. In some embodiments, one or more of the components are attached or configured in a deployment state, such as that shown in Figure 3, and the components are then compressed or collapsed and transported to a target implantation location. In other embodiments, components can be individually compressed or collapsed and transported to a target implantation location.

Figures 16-1 through 16-5 illustrate an example procedure for implanting a device in a patient at a target implantation location in accordance with one or more embodiments of the present disclosure. In some embodiments, the device can be implanted within a patient using a catheter-based procedure. For instance, the implantation procedure can be similar to a transcatheter aortic valve replacement (TAVR) or transcatheter aortic valve implantation (TAVI) procedure that uses transcaval access.

As illustrated in Figure 16-1, a guidewire 1680 is introduced into an inferior vena cava 1620 and then into an aorta 1630 around renal veins 1625 and artery 1635. That is, the guidewire 1680 travels up through the inferior vena cava 1620, through a wall of the inferior vena cava 1620 that is adjacent to a wall of the aorta 1630 and through the wall of the aorta 1630. As shown in Figure 16-1, the inferior vena cava 1620 is substantially adjacent to the aorta 1630 at a location where the guidewire 1680 crosses over from the inferior vena cava 1620 into the aorta 1630. In some embodiments, the guidewire 1680 enters a patient through a femoral vein. Although the guidewire 1680 can enter a patient at any location.

As illustrated in Figure 16-2, a first expandable chamber structure 1670 can then be introduced into the aorta 1630 using the guidewire 1680 and/or a catheter (not illustrated). For example, the first expandable chamber structure 1670 can be implemented with a wire-frame structure (e.g., a stent structure) that is compressed or collapsed to implant the first expandable chamber structure 1670 in the aorta 1630 near the renal veins 1625. Figure 16-2 illustrates the first expandable chamber structure 1670 in a compressed or collapsed state. As illustrated in Figure 16-3, once the first expandable chamber structure 1670 is located within the aorta 1630, the first expandable chamber structure 1670 is expanded to a deployed state (e.g., the stent structure of the first expandable chamber structure 1670 is expanded), such as a state in which the first expandable chamber structure 1670 functions to pump blood.

As illustrated in Figure 16-4, a catheter can then be used with the guidewire 1680 to introduce a second expandable chamber structure 1650 into the inferior vena cava 1620. For example, the second expandable chamber structure 1650 can be implemented with a wire-frame structure (e.g., a stent structure) that is compressed or collapsed to implant the second expandable chamber structure 1650 in the inferior vena cava 1620 at or near the renal veins 1625. Figure 16-4 illustrates the second expandable chamber structure 1650 in a compressed or collapsed state. The second expandable chamber structure 1650 can introduced into the inferior vena cava 1620 along with other components, such as an anchor structure and/or conduit structure(s). In some embodiments, the second expandable chamber structure 1650 is implanted with a group of components, such as those illustrated in the example of Figure 3, connected to each other in a configuration in which the second expandable chamber structure 1650 will be used. Once connected in such configuration, the components are compressed or collapsed as a group and then implanted within the inferior vena cava 1620.

After implantation at or near the renal veins 1625, the second expandable chamber structure 1650 is expanded to a deployed state, such as a state in which the second expandable chamber structure 1650 functions to pump blood. The second expandable chamber structure 1650 can be connected to the first expandable chamber structure 1670 via the conduit structure 1660 at various times, such as whenever the second expandable chamber structure 1650 is located within proximity (e.g., a predetermined distance) to the first expandable chamber structure 1670 in a collapsed state or expanded state. In some embodiments, the conduit structure 1660 is implanted along with the first expandable chamber structure 1670 and/or the second expandable chamber structure 1650, or is separately implanted within a wall of the inferior vena cava 1620 and a wall of the aorta 1630. Figure 16-5 illustrates the second expandable chamber structure 1650 and the first expandable chamber structure 1670 in a connected and deployed state, wherein the first expandable chamber structure 1670 and the second expandable chamber structure 1650 function to pump blood within the inferior vena cava 1620. For example, the first expandable chamber structure 1670 and the second expandable chamber structure 1650 can operate in cooperation to draw blood from the renal veins 1625 and pump the blood up through the inferior vena cava 1620 back to a heart (not illustrated).

Figure 17 illustrates an example process 1700 for implanting a vessel pump within one or more vessels of a patient in accordance with one or more embodiments of the present disclosure. The process 1700 can be performed to increase diastolic pressure, which can include pressure in the right atrium, pressure in the left ventricle, pressure in the inferior vena cava (or any portion of the ventricular system including one or more renal veins), or other pressure associated with the diastolic phase of the cardiac cycle (or any other phase of the cardiac cycle). In some embodiments, the process 1700 can be performed to implant the vessel pump within a first fluid vessel, such as an aorta, and a second fluid vessel, such as an inferior vena cava. For ease of discussion, many embodiments discussed below may refer to the aorta and the inferior vena cava.

At 1702, a guidewire can be advanced through a wall of the second fluid vessel and/or a wall of the first fluid vessel. For example, the guidewire can be advanced through the inferior vena cava to a location that is within a distance to a renal vein in the inferior vena cava. In some embodiments, the guidewire can enter the patient through a femoral vein. However, the guidewire can enter the patient at any location and travel through the inferior vena cava (or another fluid vessel) to a target location of implantation.

At 1704, a first expandable chamber structure can be deployed in the first fluid vessel of the patient. For example, the first expandable chamber structure can be deployed in the aorta of the patient by advancing, using the guidewire, a delivery catheter through the inferior vena cava into the aorta and deploying the first expandable chamber structure, which is connected to the delivery catheter, into the aorta.

At 1706, a second expandable chamber structure can be deployed in the second fluid vessel of the patient. For example, the second expandable chamber structure can be deployed in the inferior vena cava of the patient by advancing, using the guidewire or another guidewire, the delivery catheter or another delivery catheter through the inferior vena cava to the target location of implantation and deploying the second expandable chamber structure, which is connected to a delivery catheter, into the inferior vena cava. The second expandable chamber structure can be connected to the first expandable chamber structure with a conduit that extends through a wall in the aorta and a wall in the inferior vena cava. Once connected, the second expandable chamber structure can be in fluid communication with the first expandable chamber structure. In some embodiments, the second expandable chamber structure can be deployed in the inferior vena cava to axially overlap with at least a portion of an input of the renal vein into the inferior vena cava.

At 1708, a fluid can be pumped in the first fluid vessel and/or the second fluid vessel using the first expandable chamber structure and/or the second expandable chamber structure. For example, blood can be drawn from the renal vein into a suction cavity associated with the second expandable chamber structure during diastole and pumped from the suction cavity during systole. In some embodiments, the blood can be drawn into the suction cavity by expanding the first expandable chamber structure and collapsing the second expandable chamber structure, and blood can be pumped from the suction cavity by collapsing the first expandable chamber structure and expanding the second expandable chamber structure.

### Additional Features and Embodiments

The above description of embodiments of the disclosure is not intended to be exhaustive or to limit the disclosure to the precise form disclosed above. While specific embodiments, and examples, are described above for illustrative purposes, various equivalent modifications are possible within the scope of the disclosure, as those skilled in the relevant art will recognize. For example, while processes or blocks are presented in a given order, alternative embodiments may perform routines having steps, or employ systems having blocks, in a different order, and some processes or blocks may be deleted, moved, added, subdivided, combined, and/or modified. Each of these processes or blocks may be implemented in a variety of different ways. Also, while processes or blocks are at times shown as being performed in series, these processes or blocks may instead be performed in parallel or may be performed at different times.

Certain terms of location are used herein with respect to the various disclosed embodiments. Although certain spatially relative terms, such as "outer," "inner," "upper," "lower," "below," "above," "vertical," "horizontal," "top," "bottom," and similar terms are used herein to describe a spatial relationship of one device/element or anatomical structure relative to another device/element or anatomical structure, it is understood that these terms are used herein for ease of description to describe the positional relationship between element(s)/structures(s), as illustrated in the drawings. Spatially relative terms are intended to encompass different orientations of the element(s)/structures(s), in use or operation, in addition to the orientations depicted in the drawings. For example, an element/structure described as "above" another element/structure may represent a position that is below or beside such other element/structure with respect to alternate orientations of the subject patient or element/structure, and vice-versa.

Conditional language used herein, such as, among others, "can," "could," "might," "may," "e.g.," and the like, unless specifically stated otherwise, or otherwise understood within the context as used, is intended in its ordinary sense and is generally intended to convey that certain embodiments include, while other embodiments do not include, certain features, elements and/or steps. Thus, such conditional language is not generally intended to imply that features, elements and/or steps are in any way required for one or more embodiments or that one or more embodiments necessarily include logic for deciding, with or without author input or prompting, whether these features, elements and/or steps are included or are to be performed in any particular embodiment.

It should be understood that certain ordinal terms (e.g., "first" or "second") may be provided for ease of reference and do not necessarily imply physical characteristics or ordering. Therefore, as used herein, an ordinal term (e.g., "first," "second," "third," etc.) used to modify an element, such as a structure, a component, an operation, etc., does not necessarily indicate priority or order of the element with respect to any other element, but rather may generally distinguish the element from another element having a similar or identical name (but for use of the ordinal term). In addition, as used herein, indefinite articles ("a" and "an") may indicate "one or more" rather than "one." Further, an operation performed "based on" a condition or event may also be performed based on one or more other conditions or events not explicitly recited. In some contexts, description of an operation or event as occurring or being performed "based on," or "based at least in part on," a stated event or condition can be interpreted as being triggered by or performed in response to the stated event or condition.

With respect to the various methods (not claimed) and processes (not claimed) disclosed herein, although certain orders of operations or steps are illustrated and/or described, it should be understood that the various steps and operations shown and described may be performed in any suitable or desirable temporal order. Furthermore, any of the illustrated and/or described operations or steps may be omitted from any given method or process, and the illustrated/described methods and processes may include additional operations or steps not explicitly illustrated or described.

Unless the context clearly requires otherwise, throughout the description and the claims, the terms "comprise," "comprising," "have," "having," "include," "including," and the like are to be construed in an open and inclusive sense, as opposed to a closed, exclusive, or exhaustive sense; that is to say, in the sense of "including, but not limited to."

The word "coupled", as generally used herein, refers to two or more elements that may be physically, mechanically, and/or electrically connected or otherwise associated, whether directly or indirectly (e.g., via one or more intermediate elements, components, and/or devices. Additionally, the words "herein," "above," "below," and words of similar import, when used in this application, shall refer to this application as a whole, and not to any particular portions of the present disclosure. Where the context permits, words in present disclosure using the singular or plural number may also include the plural or singular number, respectively.

The word "or" in reference to a list of two or more items, that word covers all of the following interpretations of the word: any of the items in the list, all of the items in the list, and any combination of the items in the list. Furthermore, as used herein, the term "and/or" used between elements (e.g., between the last two of a list of elements) means any one or more of the referenced/related elements. For example, the phrase "A, B, and/or C" means "A," "B," "C," "A and B," "A and C," "B and C," or "A, B, and C."

As may be used herein, the terms "substantially" and "approximately" provides an industry-accepted tolerance for its corresponding term and/or relativity between items. For some industries, an industry-accepted tolerance is less than one percent, while for other industries, the industry-accepted tolerance may be 10 percent or more. Other examples of industry-accepted tolerances range from less than one percent to fifty percent. Industry-accepted tolerances correspond to, but are not limited to, component values, integrated circuit process variations, temperature variations, rise and fall times, thermal noise, dimensions, signaling errors, dropped packets, temperatures, pressures, material compositions, and/or performance metrics. Within an industry, tolerance variances of accepted tolerances may be more or less than a percentage level (e.g., dimension tolerance of less than approximately +/-1%). Some relativity between items may range from a difference of less than a percentage level to a few percent. Other relativity between items may range from a difference of a few percent to magnitude of differences.

One or more embodiments have been described above with the aid of method steps illustrating the performance of specified functions and relationships thereof. The boundaries and sequence of these functional building blocks and method steps have been arbitrarily defined herein for convenience of description. Alternate boundaries and sequences can be defined so long as the specified functions and relationships are appropriately performed. Further, the boundaries of these functional building blocks have been arbitrarily defined for convenience of description. Alternate boundaries could be defined as long as the certain significant functions are appropriately performed. Similarly, flow diagram blocks may also have been arbitrarily defined herein to illustrate certain significant functionality.

To the extent used, the flow diagram block boundaries and sequence could have been defined otherwise and still perform the certain significant functionality. One of average skill in the art will also recognize that the functional building blocks, and other illustrative blocks, modules and components herein, can be implemented as illustrated or by discrete components, application specific integrated circuits, processors executing appropriate software and the like or any combination thereof.

## Claims

1. An inter-vessel fluid pump comprising:
a first expandable chamber structure (170; 270; 370; 470; 570; 670; 770; 1370; 1470; 1570; 1670) configured to be implanted within a first fluid vessel; and
a second expandable chamber structure (355; 455; 555; 655; 755; 1355; 1455; 1555) configured to be implanted within a second fluid vessel that is adjacent to the first fluid vessel, the second expandable chamber structure extending longitudinally and being in fluid communication with the first expandable chamber structure,
wherein compression of the first expandable chamber structure causes expansion of the second expandable chamber structure.

2. The inter-vessel fluid pump of claim 1, further comprising:
a first conduit structure (351; 451; 551; 651; 851; 1051; 1551) having an hour-glass-shaped profile and a lumen that extends longitudinally; and
a second conduit structure (352; 452; 552; 652; 852; 952; 1552) extending longitudinally and including one or more holes (354; 454; 554; 654; 854; 954) that extend radially, the second conduit structure being configured to house at least a portion of the first conduit structure and to align longitudinally with the first conduit structure;
wherein the second expandable chamber structure is configured to be disposed between the first conduit structure and the second conduit structure.

3. The inter-vessel fluid pump of claim 2, further comprising:
a valve structure (356; 456; 556; 656; 1256) configured to align longitudinally with the first conduit structure and configured to be disposed between the second expandable chamber structure and the second conduit structure, the valve structure being configured to be displaced radially to obstruct the one or more holes of the second conduit structure when the second expandable chamber structure is expanded.

4. The inter-vessel fluid pump of claim 3, wherein one or more of the second expandable chamber structure and the valve structure has a tapered cylindrical shape.

5. The inter-vessel fluid pump of claim 2, wherein the first conduit structure includes one or more one-way valves disposed in at least one end of the first conduit structure.

6. The inter-vessel fluid pump of claim 5, wherein in a deployed state the first conduit structure and the second conduit structure are disposed to create a suction cavity (357; 557; 657; 857) between the first conduit structure and the second conduit structure, and wherein the second expandable chamber structure is configured to expand radially to push a fluid in the suction cavity through the one or more one-way valves.

7. The inter-vessel fluid pump of any of claims 1 to 6, wherein one or more of the first expandable chamber structure and the second expandable chamber structure includes a wire-frame structure.

8. The inter-vessel fluid pump of any of claims 1 to 7, further comprising a conduit structure (260; 360; 560; 660; 760; 1360; 1560; 1660) to connect the first expandable chamber structure to the second expandable chamber structure with the fluid.

9. The inter-vessel fluid pump of any of claims 1 to 8, wherein the first expandable chamber structure and/or the second expandable chamber structure are implemented as a compliant or semi-compliant balloon.

10. A vessel pump comprising:
a first expandable chamber structure (170; 270; 370; 470; 570; 670; 770; 1370; 1470; 1570; 1670) configured to be implanted within a first fluid vessel;
a second expandable chamber structure (355; 455; 555; 655; 755; 1355; 1455; 1555) configured to be in fluid communication with the first expandable chamber structure; and
an anchor structure (150; 250; 350; 850; 1650) configured to be implanted within a second fluid vessel and to house at least a portion of the second expandable chamber structure,
wherein, in a deployed state, the second expandable chamber structure is configured to expand to fill at least a portion of a suction cavity within the anchor structure.

11. The vessel pump of claim 10, wherein the first fluid vessel comprises an abdominal aorta and the second fluid vessel comprises an inferior vena cava.

12. The vessel pump of claim 10 or claim 11, wherein a cross-section of a first end of the anchor structure has a first diameter and a cross-section of a central portion of the anchor structure has a second diameter that is smaller than the first diameter.

13. The vessel pump of any of claims 10 to 12, wherein one or more of the first expandable chamber structure and the second expandable chamber structure comprise a compliant balloon.

14. The vessel pump of any of claims 10 to 13, wherein the anchor structure includes one or more one-way valves configured to permit flow of fluid from the suction cavity into the second fluid vessel when deployed.

15. The vessel pump of any of claims 10 to 14, further comprising a valve structure (356; 556; 656) configured to be disposed within with the anchor structure and to be displaced radially to obstruct one or more holes in the anchor structure when the second expandable chamber structure is expanded.

## Patentansprüche

1. Zwischen-Gefäß-Fluidpumpe, umfassend:
eine erste expandierbare Kammerstruktur (170; 270; 370; 470; 570; 670; 770; 1370; 1470; 1570; 1670), die dazu konfiguriert ist, in einem ersten Fluidgefäß implantiert zu werden; und
eine zweite expandierbare Kammerstruktur (355; 455; 555; 655; 755; 1355; 1455; 1555), die dazu konfiguriert ist, in einem zweiten Fluidgefäß implantiert zu werden, das an das erste Fluidgefäß angrenzt, wobei sich die zweite expandierbare Kammerstruktur in Längsrichtung erstreckt und mit der ersten expandierbaren Kammerstruktur in Fluidverbindung steht,
wobei eine Kompression der ersten expandierbaren Kammerstruktur eine Expansion der zweiten expandierbaren Kammerstruktur bewirkt.

2. Zwischen-Gefäß-Fluidpumpe nach Anspruch 1, ferner umfassend:
eine erste Kanalstruktur (351; 451; 551; 651; 851; 1051; 1551), die ein sanduhrförmiges Profil und ein Lumen aufweist, das sich in Längsrichtung erstreckt; und
eine zweite Kanalstruktur (352; 452; 552; 552; 852; 952; 1552), die sich in Längsrichtung erstreckt und ein oder mehrere Löcher (354; 454; 554; 654; 854; 954) enthält, die sich radial erstrecken, wobei die zweite Kanalstruktur dazu konfiguriert ist, wenigstens einen Abschnitt der ersten Kanalstruktur aufzunehmen und sich in Längsrichtung auf die erste Kanalstruktur auszurichten;
wobei die zweite expandierbare Kammerstruktur dazu konfiguriert ist, zwischen der ersten Kanalstruktur und der zweiten Kanalstruktur angeordnet zu sein.

3. Zwischen-Gefäß-Fluidpumpe nach Anspruch 2, ferner umfassend:
eine Ventilstruktur (356; 456; 556; 656; 1256), die dazu konfiguriert ist, sich in Längsrichtung auf die erste Kanalstruktur auszurichten, und die dazu konfiguriert ist, zwischen der zweiten expandierbaren Kammerstruktur und der zweiten Kanalstruktur angeordnet zu sein, wobei die Ventilstruktur dazu konfiguriert ist, radial verschoben zu werden, um das eine oder die mehreren Löcher der zweiten Kanalstruktur zu blockieren, wenn die zweite expandierbare Kammerstruktur expandiert wird.

4. Zwischen-Gefäß-Fluidpumpe nach Anspruch 3, wobei die zweite expandierbare Kammerstruktur und/oder die Ventilstruktur eine sich verjüngende zylindrische Form aufweist.

5. Zwischen-Gefäß-Fluidpumpe nach Anspruch 2, wobei die erste Kanalstruktur ein oder mehrere Einwegventile enthält, die in wenigstens einem Ende der ersten Kanalstruktur angeordnet sind.

6. Zwischen-Gefäß-Fluidpumpe nach Anspruch 5, wobei in einem eingesetzten Zustand die erste Kanalstruktur und die zweite Kanalstruktur so angeordnet sind, dass sie einen Ansaughohlraum (357; 557; 657; 857) zwischen der ersten Kanalstruktur und der zweiten Kanalstruktur bilden, und wobei die zweite expandierbare Kammerstruktur dazu konfiguriert ist, radial zu expandieren, um ein Fluid in dem Ansaughohlraum durch das eine oder die mehreren Einwegventile zu drücken.

7. Zwischen-Gefäß-Fluidpumpe nach einem der Ansprüche 1 bis 6, wobei die erste expandierbare Kammerstruktur und/oder die zweite expandierbare Kammerstruktur eine Drahtrahmenstruktur enthält.

8. Zwischen-Gefäß-Fluidpumpe nach einem der Ansprüche 1 bis 7, die ferner eine Kanalstruktur (260; 360; 560; 660; 760; 1360; 1560; 1660) umfasst, um die erste expandierbare Kammerstruktur mit der zweiten expandierbaren Kammerstruktur mittels des Fluids zu verbinden.

9. Zwischen-Gefäß-Fluidpumpe nach einem der Ansprüche 1 bis 8, wobei die erste expandierbare Kammerstruktur und/oder die zweite expandierbare Kammerstruktur als ein nachgiebiger oder halbnachgiebiger Ballon implementiert ist.

10. Gefäßpumpe, umfassend:
eine erste expandierbare Kammerstruktur (170; 270; 370; 470; 570; 670; 770; 1370; 1470; 1570; 1670), die dazu konfiguriert ist, in ein erstes Fluidgefäß implantiert zu werden;
eine zweite expandierbare Kammerstruktur (355; 455; 555; 655; 755; 1355; 1455; 1555), die dazu konfiguriert ist, mit der ersten expandierbaren Kammerstruktur in Fluidverbindung zu stehen; und
eine Ankerstruktur (150; 250; 350; 350; 1650), die dazu konfiguriert ist, in ein zweites Fluidgefäß implantiert zu werden und wenigstens einen Abschnitt der zweiten expandierbaren Kammerstruktur aufzunehmen,
wobei in einem eingesetzten Zustand die zweite expandierbare Kammerstruktur dazu konfiguriert ist, zu expandieren, um wenigstens einen Abschnitt eines Ansaughohlraums innerhalb der Ankerstruktur zu füllen.

11. Gefäßpumpe nach Anspruch 10, wobei das erste Fluidgefäß eine abdominale Aorta umfasst und das zweite Fluidgefäß eine Vena cava inferior umfasst.

12. Gefäßpumpe nach Anspruch 10 oder Anspruch 11, wobei ein Querschnitt eines ersten Endes der Ankerstruktur einen ersten Durchmesser aufweist und ein Querschnitt eines mittleren Abschnitts der Ankerstruktur einen zweiten Durchmesser aufweist, der kleiner ist als der erste Durchmesser.

13. Gefäßpumpe nach einem der Ansprüche 10 bis 12, wobei die erste expandierbare Kammerstruktur und/oder die zweite expandierbare Kammerstruktur einen nachgiebigen Ballon umfasst.

14. Gefäßpumpe nach einem der Ansprüche 10 bis 13, wobei die Ankerstruktur ein oder mehrere Einwegventile enthält, die dazu konfiguriert sind, nach dem Einsetzen den Durchfluss von Fluid aus dem Ansaughohlraum in das zweite Fluidgefäß zu ermöglichen.

15. Gefäßpumpe nach einem der Ansprüche 10 bis 14, die ferner eine Ventilstruktur (356; 556; 656) umfasst, die dazu konfiguriert ist, innerhalb der Ankerstruktur angeordnet zu werden und radial verschoben zu werden, um ein oder mehrere Löcher in der Ankerstruktur zu blockieren, wenn die zweite expandierbare Kammerstruktur expandiert wird.

## Revendications

1. Pompe à fluide intra-vaisseau comprenant :
une première structure de chambre expansible (170 ; 270 ; 370 ; 470 ; 570 ; 670 ; 770 ; 1370 ; 1470 ; 1570 ; 1670) conçue pour être implantée à l'intérieur d'un premier vaisseau à fluide ; et
une seconde structure de chambre expansible (355 ; 455 ; 555 ; 655 ; 755 ; 1355 ; 1455 ; 1555) conçue pour être implantée à l'intérieur d'un second vaisseau à fluide qui est adjacent au premier vaisseau à fluide, la seconde structure de chambre expansible s'étendant longitudinalement et étant en communication fluidique avec la première structure de chambre expansible,
la compression de la première structure de chambre expansible provoquant la dilatation de la seconde structure de chambre expansible.

2. Pompe à fluide intra-vaisseau selon la revendication 1, comprenant en outre :
une première structure de conduit (351 ; 451 ; 551 ; 651 ; 851 ; 1051 ; 1551) présentant un profil en forme de sablier et une lumière qui s'étend longitudinalement ; et
une seconde structure de conduit (352 ; 452 ; 552 ; 652 ; 852 ; 952 ; 1552) s'étendant longitudinalement et comprenant un ou plusieurs trous (354 ; 454 ; 554 ; 654 ; 854 ; 954) qui s'étendent radialement, la seconde structure de conduit étant conçue pour loger au moins une partie de la première structure de conduit et pour s'aligner longitudinalement avec la première structure de conduit ;
la seconde structure de chambre expansible étant conçue pour être disposée entre la première structure de conduit et la seconde structure de conduit.

3. Pompe à fluide intra-vaisseau selon la revendication 2, comprenant en outre :
une structure de valve (356 ; 456 ; 556 ; 656 ; 1256) conçue pour s'aligner longitudinalement avec la première structure de conduit et conçue pour être disposée entre la seconde structure de chambre expansible et la seconde structure de conduit, la structure de valve étant conçue pour être déplacée radialement afin d'obstruer ledit un ou lesdits plusieurs trous de la seconde structure de conduit lorsque la seconde structure de chambre expansible est dilatée.

4. Pompe à fluide intra-vaisseau selon la revendication 3, un ou plusieurs éléments parmi la seconde structure de chambre expansible et la structure de soupape présentant une forme cylindrique effilée.

5. Pompe à fluide intra-vaisseau selon la revendication 2, la première structure de conduit comprenant une ou plusieurs valves unidirectionnelles disposées dans au moins une extrémité de la première structure de conduit.

6. Pompe à fluide intra-vaisseau selon la revendication 5, où, dans un état déployé, la première structure de conduit et la seconde structure de conduit sont disposées afin de créer une cavité d'aspiration (357 ; 557 ; 657 ; 857) entre la première structure de conduit et la seconde structure de conduit et la seconde structure de chambre expansible étant conçue pour se dilater radialement afin de pousser un fluide dans la cavité d'aspiration à travers ladite une ou lesdites plusieurs valves unidirectionnelles.

7. Pompe à fluide intra-vaisseau selon l'une quelconque des revendications 1 à 6, un ou plusieurs éléments parmi la première structure de chambre expansible et la seconde structure de chambre expansible comprenant une structure de cadre grillagé.

8. Pompe à fluide intra-vaisseau selon l'une quelconque des revendications 1 à 7, comprenant en outre une structure de conduit (260 ; 360 ; 560 ; 660 ; 760 ; 1360 ; 1560 ; 1660) destinée à relier la première structure de chambre expansible à la seconde structure de chambre expansible par le fluide.

9. Pompe à fluide intra-vaisseau selon l'une quelconque des revendications 1 à 8, la première structure de chambre expansible et/ou la seconde structure de chambre expansible étant exécutée(s) sous la forme d'un ballonnet souple ou semi-souple.

10. Pompe à vaisseau comprenant :
une première structure de chambre expansible (170 ; 270 ; 370 ; 470 ; 570 ; 670 ; 770 ; 1370 ; 1470 ; 1570 ; 1670) conçue pour être implantée à l'intérieur d'un premier vaisseau à fluide ;
une seconde structure de chambre expansible (355 ; 455 ; 555 ; 655 ; 755 ; 1355 ; 1455 ; 1555) conçue pour être en communication fluidique avec la première structure de chambre expansible ; et
une structure d'ancrage (150 ; 250 ; 350 ; 850 ; 1650) conçue pour être implantée à l'intérieur d'un second vaisseau à fluide et pour loger au moins une partie de la seconde structure de chambre expansible,
où, dans un état déployé, la seconde structure de chambre expansible est conçue pour se dilater afin de remplir au moins une partie d'une cavité d'aspiration à l'intérieur de la structure d'ancrage.

11. Pompe à vaisseau selon la revendication 10, le premier vaisseau à fluide comprenant une aorte abdominale et le second vaisseau à fluide comprenant une veine cave inférieure.

12. Pompe à vaisseau selon la revendication 10 ou la revendication 11, une section transversale d'une première extrémité de la structure d'ancrage présentant un premier diamètre et une section transversale d'une partie centrale de la structure d'ancrage présentant un second diamètre qui est plus petit que le premier diamètre.

13. Pompe à vaisseau selon l'une quelconque des revendications 10 à 12, un ou plusieurs éléments parmi la première structure de chambre expansible et la seconde structure de chambre expansible comprenant un ballonnet souple.

14. Pompe à vaisseau selon l'une quelconque des revendications 10 à 13, la structure d'ancrage comprenant une ou plusieurs valves unidirectionnelles conçues pour permettre l'écoulement de fluide à partir de la cavité d'aspiration dans le second vaisseau à fluide en cas de déploiement.

15. Pompe à vaisseau selon l'une quelconque des revendications 10 à 14, comprenant en outre une structure de valve (356 ; 556 ; 656) conçue pour être disposée à l'intérieur de la structure d'ancrage et pour être déplacée radialement afin d'obstruer un ou plusieurs trous dans la structure d'ancrage lorsque la seconde structure de chambre expansible est dilatée.
